# EUROPEAN PATENT SPECIFICATION

(11) **EP 1 287 137 B1**
(45) Date of publication and mention of the grant of the patent: **25.05.2005**
(21) Application number: 01949336.0
(22) Date of filing: 11.05.2001
(51) Int. Cl.: C12N 15/12, C12N 15/62, C07K 14/705, C12Q 1/68, G01N 33/68, A61K 31/7088, A61K 39/395

(54) **REGULATION OF HUMAN $g(a)1A?ADRENERGIC RECEPTOR-LIKE G PROTEIN-COUPLED RECEPTOR**
REGULATION DES MENSCHLICHEN ALPHA-1A-ADRENERGEN REZEPTOR-ÄHNLICHEN G-PROTEIN GEKOPPELTEN REZEPTORS
REGULATION DU RECEPTEUR COUPLE AUX PROTEINES G DU TYPE RECEPTEUR ADRENERGIQUE $G(A)1A HUMAIN

(30) Priority: 15.05.2000 US 204145 P; 04.12.2000 US 250505 P
(43) Date of publication of application: 05.03.2003
(73) Proprietor: Bayer HealthCare AG, 51368 Leverkusen (DE)
(72) Inventor: RAMAKRISHNAN, Shyam, Brighton, MA 02135 (US)
(86) International application number: PCT/EP2001/005383
(87) International publication number: WO 2001/088126

(56) References cited:
- WO-A-98/46620
- US-A- 5 994 506
- DATABASE EMBL [Online] embl; AC016468, 1 December 1999 (1999-12-01) BIRREN B. ET AL.,: "homo sapiens clone RP11-14N15" XP002196601
- LEE D K ET AL: "Cloning and characterization of additional members of the G protein-coupled receptor family" BIOCHIMICA ET BIOPHYSICA ACTA. GENE STRUCTURE AND EXPRESSION, ELSEVIER, AMSTERDAM, NL, vol. 1490, no. 3, 29 February 2000 (2000-02-29), pages 311-323, XP004275600 ISSN: 0167-4781
- LEE D K ET AL: "Discovery and mapping of ten novel G protein-coupled receptor genes" GENE: AN INTERNATIONAL JOURNAL ON GENES AND GENOMES, ELSEVIER SCIENCE PUBLISHERS, BARKING, GB, vol. 275, no. 1, 5 September 2001 (2001-09-05), pages 83-91, XP004307114 ISSN: 0378-1119
- DATABASE EMBL [Online] embl; AL356783, 24 May 2000 (2000-05-24) HEATH P.: "human sequence clone RP13-13L21" XP002196602

### SEQUENCE LISTING

<110> Bayer AG
<120> REGULATION OF HUMAN alphalA ADRENERGIC RECEPTOR-LIKE G PROTEIN-COUPLED RECEPTOR
<130> Lio062 Foreign Countries
<140>
   <141>
<150> US 60/204,145
   <151> 2000-05-15
<150> US 60/250,505
   <151> 2000-12-04
<160> 5
<170> PatentIn Ver. 2.1
<210> 1
   <211> 1920
   <212> DNA
   <213> Homo sapiens
<220>
   <221> misc_feature
   <222> (237)..(1763)
   <223> coding sequence
<400> 1
   aaaggagcca tagaagctgc ccgcactggg gatggagccg tgcggaaacc cggggtaggg 60
   ggtcctgcag cgtccttgct gggcgcggag gcttctcccc ttgacgggtg actaactctg 120
   cctgcgtgtt tcttttgtca ccagcatagg cactgagtgc ggtctgtgca cccctttgcc 180
   acccaccggt gccggcactg agcctgcaac ctgtctcacg ccctctggct gttgccatga 240
   cgtccacctg caccaacagc acgcgcgaga gtaacagcag ccacacgtgc atgcccctct 300
   ccaaaatgcc catcagcctg gcccacggca tcatccgctc aaccgtgctg gttatcttcc 360
   tcgccgcctc tttcgtcggc aacatagtgc tggcgctagt gttgcagcgc aagccgcagc 420
   tgctgcaggt gaccaaccgt tttatcttta acctcctcgt caccgacctg ctgcagattt 480
   cgctcgtggc cccctgggtg gtggccacct ctgtgcctct cttctggccc ctcaacagcc 540
   acttctgcac ggccctggtt agcctcaccc acctgttcgc cttcgccagc gtcaacacca 600
   ttgtcgtggt gtcagtggat cgctacttgt ccatcatcca ccctctctcc tacccgtcca 660
   agatgaccca gcgccgcggt tacctgctcc tctatggcac ctggattgtg gccatcctgc 720
   agagcactcc tccactctac ggctggggcc aggctgcctt tgatgagcgc aatgctctct 780
   gctccatgat ctggggggcc agccccagct acactattct cagcgtggtg tccttcatcg 840
   tcattccact gattgtcatg attgcctgct actccgtggt gttctgtgca gcccggaggc 900
   agcatgctct gctgtacaat gtcaagagac acagcttgga agtgcgagtc aaggactgtg 960
   tggagaatga ggatgaagag ggagcagaga agaaggagga gttccaggat gagagtgagt 1020
   ttcgccgcca gcatgaaggt gaggtcaagg ccaaggaggg cagaatggaa gccaaggacg 1080
   gcagcctgaa ggccaaggaa ggaagcacgg ggaccagtga gagtagtgta gaggccaggg 1140
   gcagcgagga ggtcagagag agcagcacgg tggccagcga cggcagcatg gagggtaagg 1200
   aaggcagcac caaagttgag gagaacagca tgaaggcaga caagggtcgc acagaggtca 1260
   accagtgcag cattgacttg ggtgaagatg acatggagtt tggtgaagac gacatcaatt 1320
   tcagtgagga tgacgtcgag gcagtgaaca tcccggagag cctcccaccc agtcgtcgta 1380
   acagcaacag caaccctcct ctgcccaggt gctaccagtg caaagctgct aaagtgatct 1440
   tcatcatcat tttctcctat gtgctatccc tggggcccta ctgcttttta gcagtcctgg 1500
   ccgtgtgggt ggatgtcgaa acccaggtac cccagtgggt gatcaccata atcatctggc 1560
   ttttcttcct gcagtgctgc atccacccct atgtctatgg ctacatgcac aagaccatta 1620
   agaaggaaat ccaggacatg ctgaagaagt tcttctgcaa ggaaaagccc ccgaaagaag 1680
   atagccaccc agacctgccc ggaacagagg gtgggactga aggcaagatt gtcccttcct 1740
   acgattctgc tacttttcct tgaagttagt tctaaggcaa accttgaact gtccataaca 1800
   cgagaaacaa gaggagattt cttttcaatg gacccacaat tcattaatgc caaaccatac 1860
   catttcaggc aaaggtgttg cacacacatg ctcttcacca caaggtagat aaatatatag 1920
<210> 2
   <211> 1527
   <212> DNA
   <213> Homo sapiens
<400> 2
   atgacgtcca cctgcaccaa cagcacgcgc gagagtaaca gcagccacac gtgcatgccc 60
   ctctccaaaa tgcccatcag cctggcccac ggcatcatcc gctcaaccgt gctggttatc 120
   ttcctcgccg cctctttcgt cggcaacata gtgctggcgc tagtgttgca gcgcaagccg 180
   cagctgctgc aggtgaccaa ccgttttatc tttaacctcc tcgtcaccga cctgctgcag 240
   atttcgctcg tggccccctg ggtggtggcc acctctgtgc ctctcttctg gcccctcaac 300
   agccacttct gcacggccct ggttagcctc acccacctgt tcgccttcgc cagcgtcaac 360
   accattgtcg tggtgtcagt ggatcgctac ttgtccatca tccaccctct ctcctacccg 420
   tccaagatga cccagcgccg cggttacctg ctcctctatg gcacctggat tgtggccatc 480
   ctgcagagca ctcctccact ctacggctgg ggccaggctg cctttgatga gcgcaatgct 540
   ctctgctcca tgatctgggg ggccagcccc agctacacta ttctcagcgt ggtgtccttc 600
   atcgtcattc cactgattgt catgattgcc tgctactccg tggtgttctg tgcagcccgg 660
   aggcagcatg ctctgctgta caatgtcaag agacacagct tggaagtgcg agtcaaggac 720
   tgtgtggaga atgaggatga agagggagca gagaagaagg aggagttcca ggatgagagt 780
   gagtttcgcc gccagcatga aggtgaggtc aaggccaagg agggcagaat ggaagccaag 840
   gacggcagcc tgaaggccaa ggaaggaagc acggggacca gtgagagtag tgtagaggcc 900
   aggggcagcg aggaggtcag agagagcagc acggtggcca gcgacggcag catggagggt 960
   aaggaaggca gcaccaaagt tgaggagaac agcatgaagg cagacaaggg tcgcacagag 1020
   gtcaaccagt gcagcattga cttgggtgaa gatgacatgg agtttggtga agacgacatc 1080
   aatttcagtg aggatgacgt cgaggcagtg aacatcccgg agagcctccc acccagtcgt 1140
   cgtaacagca acagcaaccc tcctctgccc aggtgctacc agtgcaaagc tgctaaagtg 1200
   atcttcatca tcattttctc ctatgtgcta tccctggggc cctactgctt tttagcagtc 1260
   ctggccgtgt gggtggatgt cgaaacccag gtaccccagt gggtgatcac cataatcatc 1320
   tggcttttct tcctgcagtg ctgcatccac ccctatgtct atggctacat gcacaagacc 1380
   attaagaagg aaatccagga catgctgaag aagttcttct gcaaggaaaa gcccccgaaa 1440
   gaagatagcc acccagacct gcccggaaca gagggtggga ctgaaggcaa gattgtccct 1500
   tcctacgatt ctgctacttt tccttga                                    1527
<210> 3
   <211> 508
   <212> PRT
   <213> Homo sapiens
<400> 3
   Met Thr Ser Thr Cys Thr Asn Ser Thr Arg Glu Ser Asn Ser Ser His
    1               5                  10                  15
   Thr Cys Met Pro Leu Ser Lys Met Pro Ile Ser Leu Ala His Gly Ile
               20                  25                  30
   Ile Arg Ser Thr Val Leu Val Ile Phe Leu Ala Ala Ser Phe Val Gly
           35                  40                  45
   Asn Ile Val Leu Ala Leu Val Leu Gln Arg Lys Pro Gln Leu Leu Gln
       50                  55                  60
   Val Thr Asn Arg Phe Ile Phe Asn Leu Leu Val Thr Asp Leu Leu Gln
   65                  70                  75                  80
   Ile Ser Leu Val Ala Pro Trp Val Val Ala Thr Ser Val Pro Leu Phe
                   85                  90                  95
   Trp Pro Leu Asn Ser His Phe Cys Thr Ala Leu Val Ser Leu Thr His
               100                 105                 110
   Leu Phe Ala Phe Ala Ser Val Asn Thr Ile Val Val Val Ser Val Asp
           115                 120                 125
   Arg Tyr Leu Ser Ile Ile His Pro Leu Ser Tyr Pro Ser Lys Met Thr
       130                 135                 140
   Gln Arg Arg Gly Tyr Leu Leu Leu Tyr Gly Thr Trp Ile Val Ala Ile
   145                 150                 155                 160
   Leu Gln Ser Thr Pro Pro Leu Tyr Gly Trp Gly Gln Ala Ala Phe Asp
                   165                 170                 175
   Glu Arg Asn Ala Leu Cys Ser Met Ile Trp Gly Ala Ser Pro Ser Tyr
               180                 185                 190
   Thr Ile Leu Ser Val Val Ser Phe Ile Val Ile Pro Leu Ile Val Met
   195 200 205
   Ile Ala Cys Tyr Ser Val Val Phe Cys Ala Ala Arg Arg Gln His Ala
   210 215 220
   Leu Leu Tyr Asn Val Lys Arg His Ser Leu Glu Val Arg Val Lys Asp
   225 230 235 240
   Cys Val Glu Asn Glu Asp Glu Glu Gly Ala Glu Lys Lys Glu Glu Phe
   245 250 255
   Gln Asp Glu Ser Glu Phe Arg Arg Gln His Glu Gly Glu Val Lys Ala
   260 265 270
   Lys Glu Gly Arg Met Glu Ala Lys Asp Gly Ser Leu Lys Ala Lys Glu
   275 280 285
   Gly Ser Thr Gly Thr Ser Glu Ser Ser Val Glu Ala Arg Gly Ser Glu
   290 295 300
   Glu Val Arg Glu Ser Ser Thr Val Ala Ser Asp Gly Ser Met Glu Gly
   305 310 315 320
   Lys Glu Gly Ser Thr Lys Val Glu Glu Asn Ser Met Lys Ala Asp Lys
   325 330 335
   Gly Arg Thr Glu Val Asn Gln Cys Ser Ile Asp Leu Gly Glu Asp Asp
   340 345 350
   Met Glu Phe Gly Glu Asp Asp Ile Asn Phe Ser Glu Asp Asp Val Glu
   355 360 365
   Ala Val Asn Ile Pro Glu Ser Leu Pro Pro Ser Arg Arg Asn Ser Asn
   370 375 380
   Ser Asn Pro Pro Leu Pro Arg Cys Tyr Gln Cys Lys Ala Ala Lys Val
   385 390 395 400
   Ile Phe Ile Ile Ile Phe Ser Tyr Val Leu Ser Leu Gly Pro Tyr Cys
   405 410 415
   Phe Leu Ala Val Leu Ala Val Trp Val Asp Val Glu Thr Gln Val Pro
   420 425 430
   Gln Trp Val Ile Thr Ile Ile Ile Trp Leu Phe Phe Leu Gln Cys Cys
   435 440 445
   Ile His Pro Tyr Val Tyr Gly Tyr Met His Lys Thr Ile Lys Lys Glu
   450 455 460
   Ile Gln Asp Met Leu Lys Lys Phe Phe Cys Lys Glu Lys Pro Pro Lys
   465 470 475 480
   Glu Asp Ser His Pro Asp Leu Pro Gly Thr Glu Gly Gly Thr Glu Gly
   485 490 495
   Lys Ile Val Pro Ser Tyr Asp Ser Ala Thr Phe Pro
   500 505
<210> 4
   <211> 466
   <212> PRT
   <213> Homo sapiens
<400> 4
   Met Val Phe Leu Ser Gly Asn Ala Ser Asp Ser Ser Asn Cys Thr His
   1 5 10 15
   Pro Pro Ala Pro Val Asn Ile Ser Lys Ala Ile Leu Leu Gly Val Ile
   20 25 30
   Leu Gly Gly Leu Ile Leu Phe Gly Val Leu Gly Asn Ile Leu Val Ile
   35 40 45
   Leu Ser Val Ala Cys His Arg His Leu His Ser Val Thr His Tyr Tyr
   50 55 60
   Ile Val Asn Leu Ala Val Ala Asp Leu Leu Leu Thr Ser Thr Val Leu
   65 70 75 80
   Pro Phe Ser Ala Ile Phe Glu Ile Leu Gly Tyr Trp Ala Phe Gly Arg
   85 90 95
   Val Phe Cys Asn Ile Trp Ala Ala Val Asp Val Leu Cys Cys Thr Ala
   100 105 110
   Ser Ile Ile Ser Leu Cys Val Ile Ser Ile Asp Arg Tyr Ile Gly Val
   115 120 125
   Ser Tyr Pro Leu Arg Tyr Pro Thr Ile Val Thr Gln Arg Arg Gly Leu
   130 135 140
   Arg Ala Leu Leu Cys Val Trp Ala Phe Ser Leu Val Ile Ser Val Gly
   145 150 155 160
   Pro Leu Phe Gly Trp Arg Gln Pro Ala Pro Asp Asp Glu Thr Ile Cys
                   165                 170                 175
   Gln Ile Asn Glu Glu Pro Gly Tyr Val Leu Phe Ser Ala Leu Gly Ser
               180                 185                 190
   Phe Tyr Val Pro Leu Thr Ile Ile Leu Ala Met Tyr Cys Arg Val Tyr
           195                 200                 205
   Val Val Ala Lys Arg Glu Ser Arg Gly Leu Lys Ser Gly Leu Lys Thr
       210                 215                 220
   Asp Lys Ser Asp Ser Glu Gln Val Thr Leu Arg Ile His Arg Lys Asn
   225                 230                 235                 240
   Ala Pro Ala Gly Gly Ser Gly Val Ala Ser Ala Lys Asn Lys Thr His
                   245                 250                 255
   Phe Ser Val Arg Leu Leu Lys Phe Ser Arg Glu Lys Lys Ala Ala Lys
               260                 265                 270
   Thr Leu Gly Ile Val Val Gly Cys Phe Val Leu Cys Trp Leu Pro Phe
           275                 280                 285
   Phe Leu Val Met Pro Ile Gly Ser Phe Phe Pro Asp Phe Lys Pro Pro
       290                 295                 300
   Glu Thr Val Phe Lys Ile Val Phe Trp Leu Gly Tyr Leu Asn Ser Cys
   305                 310                 315                 320
   Ile Asn Pro Ile Ile Tyr Pro Cys Ser Ser Gln Glu Phe Lys Lys Ala
                   325                 330                 335
   Phe Gln Asn Val Leu Lys Ile Gln Cys Leu Arg Arg Lys Gln Ser Ser
               340                 345                 350
   Lys His Ala Leu Gly Tyr Thr Leu His Ala Pro Ser Gln Ala Leu Glu
           355                 360                 365
   Gly Gln His Lys Asp Met Val Arg Ile Pro Val Gly Ser Gly Glu Thr
       370                 375                 380
   Phe Tyr Lys Ile Ser Lys Thr Asp Gly Val Cys Glu Trp Lys Phe Phe
   385                 390                 395                 400
   Ser Ser Met Pro Arg Gly Ser Ala Arg Ile Thr Val Pro Lys Asp Gln
                   405                 410                 415
   Ser Ala Cys Thr Thr Ala Arg Val Arg Ser Lys Ser Phe Leu Gln Val
   420 425 430
   Cys Cys Cys Val Gly Pro Ser Thr Pro Asn Pro Gly Glu Asn His Gln
   435 440 445
   Val Pro Thr Ile Lys Ile His Thr Ile Ser Leu Ser Glu Asn Gly Glu
   450 455 460
   Glu Val
   465
<210> 5
   <211> 1194
   <212> DNA
   <213> Homo sapiens
<220>
   <221> promoter
   <222> (1)..(1076)
<220>
   <221> misc_feature
   <222> (1077)..(1079)
   <223> start codon
<400> 5
   ggagagacag aaaccacacg gagagacaca gagacaagag ggtccgagaa agacagagca 60
   agcgcacacg tgccagtgag agacagaagg agacacccag agaacccgag ggagacgcag 120
   aaacaagaga gttggagaga cacggagacg ggcagagaca cagcgaacga cattgaacag 180
   gtgcgctagg gacccacaaa gatgaactgc tggggaaata gactgagaga gaaatggaga 240
   gacaagagag cactagggaa agacggagac acacatgaac tagcgagcga cacagtgaca 300
   ggcagagaca cagagagaca gaatggatca gaaagagact gagggagaga cgggcaagac 360
   tgtgagaaag atagggagcg agagcgcaag ccctgcagag aaagtgcgct agcgaaagag 420
   acacacacag agagactgag tgacaaacag agtggaagag ataaatgaga gtaacaggaa 480
   agagacagcg tgcacgtgct atcgagagac ggagaaacag agacagacat aagtgagaga 540
   caaggatgag aaagaatcag gaaacggtga gacacataca gacacacaac ccgagagata 600
   aagaaaaaaa gtgcgctagc gagagacact ggagaagaga cagaaactga cggtcgtaaa 660
   cagacagaaa cagagagcgc acgcgcatgc gctagcgcta gcgaccaaaa ctccccggag 720
   ccagagacag tgtgagagac aagcagagaa agcgcacgca cgcacgcgcc agcaggagaa 780
   acagatgaga ggaaatcaga gccctggaga gagacaggca gacagatctg gagagtccgg 840
   aaaggagcca tagaagctgc ccgcactggg gatggagccg tgcggaaacc cggggtaggg 900
   ggtcctgcag cgtccttgct gggcgcggag gcttctcccc ttgacgggtg actaactctg 960
   cctgcgtgtt tcttttgtca ccagcatagg cactgagtgc ggtctgtgca cccctttgcc 1020
   acccaccggt gccggcactg agcctgcaac ctgtctcacg ccctctggct gttgccatga 1080
   cgtccacctg caccaacagc acgcgcgaga gtaacagcag ccacacgtgc atgcccctct 1140
   ccaaaatgcc catcagcctg gcccacggca tcatccgctc aaccgtgctg gtta       1194

## Description

### TECHNICAL FIELD OF THE INVENTION

The invention relates to the area of G-protein coupled receptors. More particularly, it relates to the area of human α₁ₐ adrenergic receptor-like G protein-coupled receptors and their regulation.

### BACKGROUND OF THE INVENTION

### G-Protein Coupled Receptors

Many medically significant biological processes are mediated by signal transduction pathways that involve G-proteins (Lefkowitz, *Nature 351,* 353-354, 1991). The family of G-protein coupled receptors (GPCR) includes receptors for hormones, neurotransmitters, growth factors, and viruses. Specific examples of GPCRs include receptors for such diverse agents as dopamine, calcitonin, adrenergic hormones, endothelin, cAMP, adenosine, acetylcholine, serotonin, histamine, thrombin, kinin, follicle stimulating hormone, opsins, endothelial differentiation gene-1, rhodopsins, odorants, cytomegalovirus, G-proteins themselves, effector proteins such as phospholipase C, adenyl cyclase, and phosphodiesterase, and actuator proteins such as protein kinase A and protein kinase C.

GPCRs possess seven conserved membrane-spanning domains connecting at least eight divergent hydrophilic loops. GPCRs (also known as 7TM receptors) have been characterized as including these seven conserved hydrophobic stretches of about 20 to 30 amino acids, connecting at least eight divergent hydrophilic loops. Most GPCRs have single conserved cysteine residues in each of the first two extracellular loops, which form disulfide bonds that are believed to stabilize functional protein structure. The seven transmembrane regions are designated as TM1, TM2, TM3, TM4, TM5, TM6, and TM7. TM3 has been implicated in signal transduction.

Phosphorylation and lipidation (palmitylation or famesylation) of cysteine residues can influence signal transduction of some GPCRs. Most GPCRs contain potential phosphorylation sites within the third cytoplasmic loop and/or the carboxy terminus. For several GPCRs, such as the β-adrenergic receptor, phosphorylation by protein kinase A and/or specific receptor kinases mediates receptor desensitization.

For some receptors, the ligand binding sites of GPCRs are believed to comprise hydrophilic sockets formed by several GPCR transmembrane domains. The hydrophilic sockets are surrounded by hydrophobic residues of the GPCRs. The hydrophilic side of each GPCR transmembrane helix is postulated to face inward and form a polar ligand binding site. TM3 has been implicated in several GPCRs as having a ligand binding site, such as the TM3 aspartate residue. TM5 serines, a TM6 asparagine, and TM6 or TM7 phenylalanines or tyrosines also are implicated in ligand binding.

GPCRs are coupled inside the cell by heterotrimeric G-proteins to various intracellular enzymes, ion channels, and transporters (see Johnson *et al*., *Endoc. Rev. 10*, 317-331, 1989). Different G-protein alpha-subunits preferentially stimulate particular effectors to modulate various biological functions in a cell. Phosphorylation of cytoplasmic residues of GPCRs is an important mechanism for the regulation of some GPCRs. For example, in one form of signal transduction, the effect of hormone binding is the activation inside the cell of the enzyme, adenylate cyclase. Enzyme activation by hormones is dependent on the presence of the nucleotide GTP. GTP also influences hormone binding. A G-protein connects the hormone receptor to adenylate cyclase. G-protein exchanges GTP for bound GDP when activated by a hormone receptor. The GTP-carrying form then binds to activated adenylate cyclase. Hydrolysis of GTP to GDP, catalyzed by the G-protein itself, returns the G-protein to its basal, inactive form. Thus, the G-protein serves a dual role, as an intermediate that relays the signal from receptor to effector, and as a clock that controls the duration of the signal.

Over the past 15 years, nearly 350 therapeutic agents targeting GPCRs receptors have been successfully introduced onto the market. This indicates that these receptors have an established, proven history as therapeutic targets. Clearly, there is an ongoing need for identification and characterization of further GPCRs which can play a role in preventing, ameliorating, or correcting dysfunctions or diseases including, but not limited to, infections such as bacterial, fungal, protozoan, and viral infections, particularly those caused by HIV viruses, pain, cancers, anorexia, bulimia, asthma, Parkinson's diseases, acute heart failure, hypotension, hypertension, urinary retention, osteoporosis, angina pectoris, myocardial infarction, ulcers, asthma, allergies, benign prostatic hypertrophy, and psychotic and neurological disorders, including anxiety, schizophrenia, manic depression, delirium, dementia, several mental retardation, and dyskinesias, such as Huntington's disease and Toiaett's syndrome.

### Adrenergic Receptors

Human adrenergic receptors are integral membrane proteins which have been classified into two broad classes, the alpha and the beta adrenergic receptors (see U.S. Patent 5,922,722). Both types mediate the action of the peripheral sympathetic nervous system upon binding of catecholamines, norepinephrine and epinephrine.

Norepinephrine is produced by adrenergic nerve endings, while epinephrine is produced by the adrenal medulla. The binding affinity of adrenergic receptors for these compounds forms one basis of the classification: alpha receptors bind norepinephrine more strongly than epinephrine and much more strongly than the synthetic compound isoproterenoL The binding affinity of these hormones is reversed for the beta receptors. In many tissues, the functional responses, such as smooth muscle contraction, induced by alpha receptor activation are opposed to responses induced by beta receptor binding.

Subsequently, the functional distinction between alpha and beta receptors was further highlighted and refined by the pharmacological characterization of these receptors from various animal and tissue sources. As a result, alpha and beta adrenergic receptors were further subdivided into α₁, α₂, α₁, and α₂ subtypes. Functional differences between α₁ and α₂ receptors have been recognized, and compounds which exhibit selective binding between these two subtypes have been developed. Thus, in WO 92/0073, the selective ability of the R(+) enantiomer of terazosin to selectively bind to adrenergic receptors of the α₁ subtype was reported. The α₁/ α₂ selectivity of this compound was disclosed as being significant because agonist stimulation of the α₂ receptors was said to inhibit secretion of epinephrine and norepinephrine, while antagonism of the α₂ receptor was said to increase secretion of these hormones. Thus, the use of non-selective α-adrenergic blockers, such as phenoxybenzamine and phentolamine, is limited by their α₂ adrenergic receptor mediated induction of increased plasma catecholamine concentration and the attendant physiological sequelae (increased heart rate and smooth muscle contraction).

For a general background on the α-adrenergic receptors, see Ruffolo, α-adrenoreceptors: Molecular Biology, Biochemistry and Pharmacology (Progress in Basic and Clinical Pharmacology series, Karger, 1991), wherein the basis of α₁/α₂ subclassification, the molecular biology, signal transduction (G-protein interaction and location of the significant site for this and ligand binding activity away from the 3'-terminus of alpha adrenergic receptors), agonist structure-activity relationships, receptor functions, and therapeutic applications for compounds exhibiting α-adrenergic receptor affinity is discussed.

The cloning, sequencing, and expression of alpha receptor subtypes from animal tissues has led to the subclassincation of the α₁ receptors into α₁ₐ (Lomasney *et al*., *J. Biol. Chem. 266,* 6365-369, 1991); rat α₁ₐ (Bruno *et al*., *BBRC 179,* 1485-90, 1991), human α₁ₐ, α_{1b} (Cotecchia *et al*., *Proc. Natl. Acad. Sci. 85*, 7159-63, 1988), hamster α_{1b} (Libert *et al*., *Science* 1989), dog α_{1b} (Ramarao *et al*., *J. Biol Chem. 267,* 21936-945, 1992), and human α_{1b}. Most recently, in a study using bovine brain, a new α_{1c} subtype was proposed (Schwinn *et al*., *J. Biol. Chem. 265,* 8183-89, 1990). Hirasawa *et al*. (*BBRC 195,* 902-09, 1993) described the cloning, functional expression. and tissue distribution of a human α_{1c} adrenergic receptor. Hoehe *et al*., *Human Mol. Genetics 1*(5) 349, 1992) noted the existence of a two-allele Pst1 restriction fragment polymorphism in the α_{1c} adrenergic receptor gene. Another study suggests that there may even be an α_{1d} receptor subtypes (see Perez *et al*., *Mol. Pharm. 40,* 876-83, 1992). Each α₁ receptor subtype exhibits its own pharmacologic and tissue specificities. Schwirm and coworkers noted that the cloned bovine α_{1c} receptor exhibited pharmacological properties proposed for the α₁ₐ subtype. Nonetheless, based on its non-expression in tissues where the α₁ₐ subtype is expressed and its sensitivity to chloroethylclonidine, the receptor was given a new designation.

The EMBL Database discloses sequence AC016468 which is *Homo sapiens* clone RP11-14N15. This clone has certain homology with polynucleotides of the invention. However, the database entry does not disclose an association of the sequence with Central Nervous System disorders.

US 5,994,506 also discloses an adrenergic receptor, its nucleotide and protein sequence, and methods of its expression. However, US 5,994,506 does not disclose an association of the sequence with Central Nervous System disorders.

WO 98/46620 and Lee et al. (2000, Biochim. Biophys. Acta, 1490: 311-323) provide further G-protein coupled receptors. However, they do not disclose an association of the polypeptides or polynucleotides of the invention with Central Nervous System disorders and Pain.

Typically, identification of active compounds is accomplished through use of animal tissues known to be enriched in adrenergic receptors. Thus, rat tissues have been used to screen for potential adrenergic receptor antagonists. However, because of species variability, compounds which appear active in animal tissue may not be active or sufficiently selective in humans. This results in substantial waste of time and effort, particularly where high volume compound screening programs are employed. There is also the danger that compounds, which might be highly effective in humans, would be missed because of their absence of appreciable affinity for the heterologous animal receptors. In this regard, it has been noted that even single amino acid changes between the sequence of biologically active proteins in one species may give rise to substantial pharmacological differences. Thus, Fong *et al*. (*J. Biol. Chem, 267,* 25668-71, 1992) showed that there are 22 divergent amino acid residues between the sequence of the human neurokinin-1 receptor and the homologous rat receptor. They further showed, in studies with mutant receptors, that substitution of only two amino acid residues was both necessary and sufficient to reproduce the rat receptor's antagonist binding affinity in the human receptor. Oksenberg *et al.* (*Nature 360,* 161-63, 1992) showed that a single amino-acid difference confers major pharmacological variation between the human and the rodent 5-hydroxytryptamine receptors. Likewise, Kuhse *et al, (Neuron 5*, 867-873, 1990) showed that a single amino-acid exchange alters the pharmacology of the neonatal rat glycine receptor subunit This difficulty and unpredictability has resulted in a need for a compound screen which will identify compounds that will be active in humans. Thus, there is a need in the art to identify human α₁-like adrenergic receptors whose activity can be regulated to provide therapeutic effects.

### SUMMARY OF THE INVENTION

It is an object of the invention to provide reagents and methods of regulating a human G protein-coupled receptor. This and other objects of the invention are provided by one or more of the embodiments described below.

One embodiment of the invention is the use of an alpha-1a-adrenergic receptor polynucleotide for the screening for substances, useful in the treatment of a Central Nervous System disorder. Another embodiment of the invention is the use of an alpha-1a-adrenergic receptor polypeptide for the screening for substances, useful in the treatment of a Central Nervous System disorder. In a preferred embodiment of the invention the Central Nervous System Disorder is Pain.

The invention thus provides a human α₁ₐ adrenergic receptor-like GPCR which can be used to identify test compounds which may act as agonists or antagonists at the receptor site. Human α₁ₐ adrenergic receptor-like GPCR and fragments thereof also are useful in raising specific antibodies which can block the receptor and effectively prevent ligand binding.

### BRIEF DESCRIPTION OF THE DRAWINGS

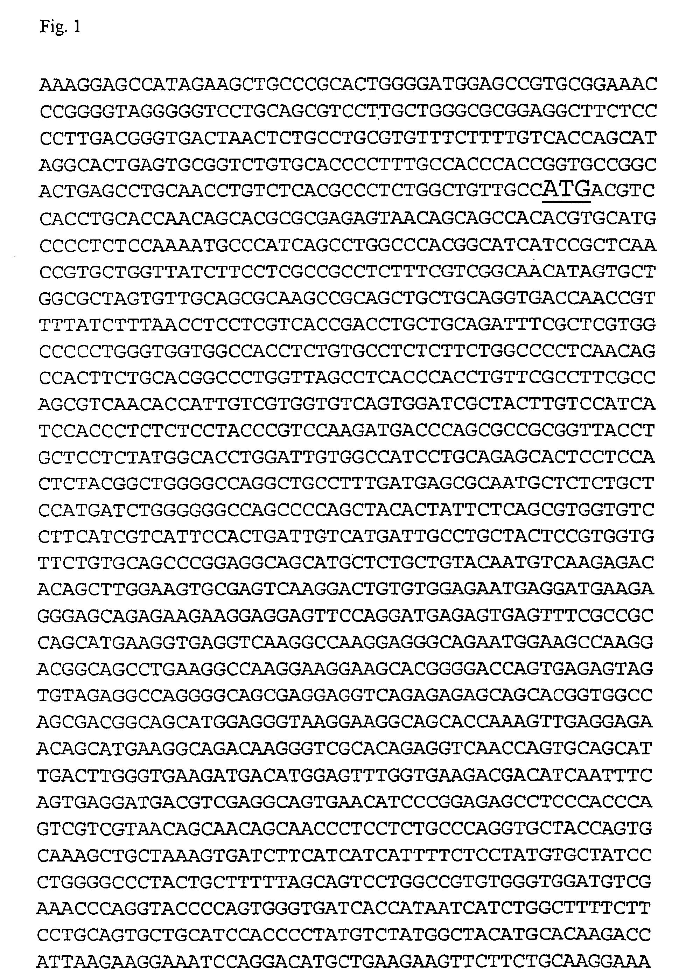

- Fig. 1: shows the human genomic DNA on chromosome 22 comprising the CDS of an α₁ₐ adrenergic receptor.

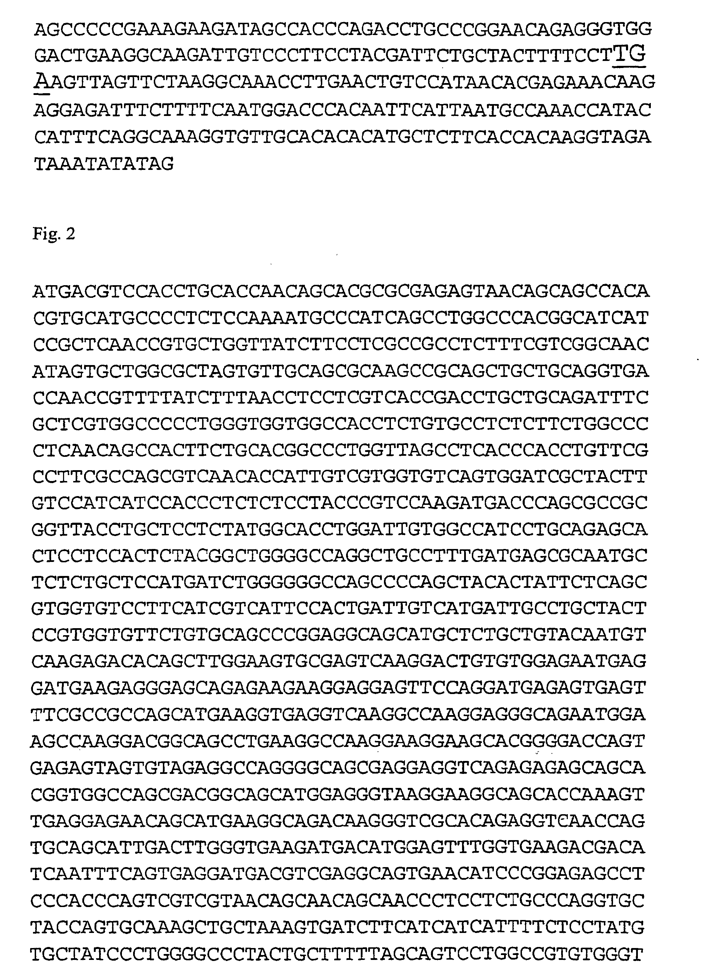

- Fig. 2: shows the DNA-sequence encoding a α₁ₐ adrenergic receptor-like GPCR polypeptide (CDS).
- Fig. 3: shows the amino acid sequence of a α₁ₐ adrenergic receptor-like GPCR polypeptide encoded by the DNA sequence of Fig. 2.
- Fig. 4: shows the amino acid sequence of *Oryctolagus cuniculus* protein polypeptide.

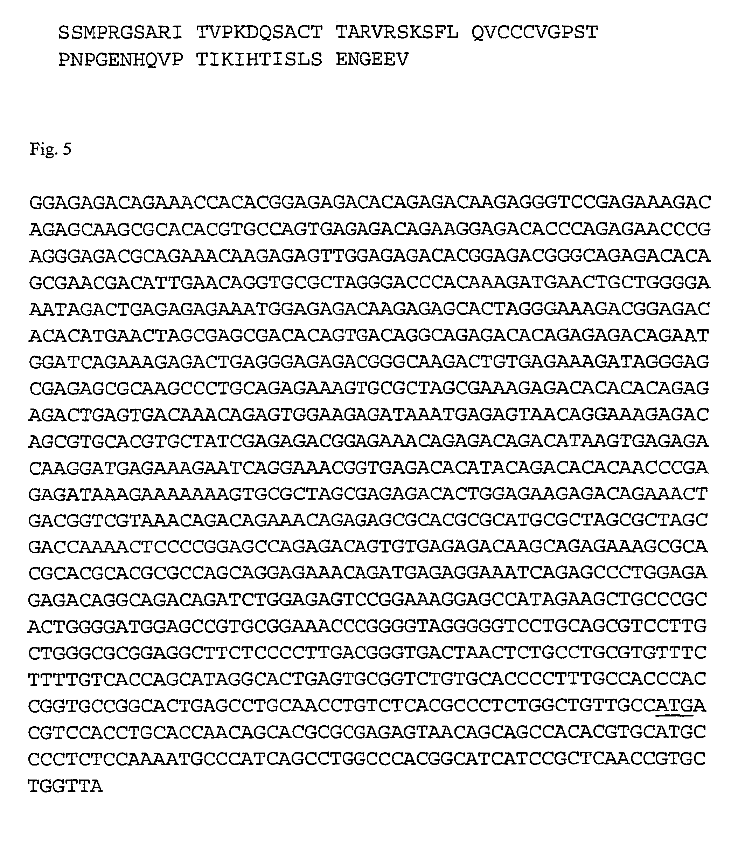

- Fig. 5: shows the promotor region and the first 118 nucleotides of the DNA-sequence of Fig. 1.
Two modules are found in the promotor region:
A. EBOX_E2FF_01
   1. Promoter module: BBOX_E2FF_01 (consists of two transacting binding domains, EBOX and E2F)
   2. The module is involved in the activation of the Epstein-Barr virus pol promoter.
   3. Reference: Liu C. et al., J. Virol. 70, 2545-2555, 1996 (MEDLINE: 8642684)
B. GATA_AP1F_01
   1. Promoter module: GATA_AP1F_01 (consists of two transacting binding domains, GATA and AP1F)
   2. The module is required for the constitutive expression of the IL-5 gene in adult T-cell leukimia cells.
   3. Reference: Yamagata T. et al., Mol. Cell. BioL 17,4272-4281, 1997 (MEDLINE: 9234684)

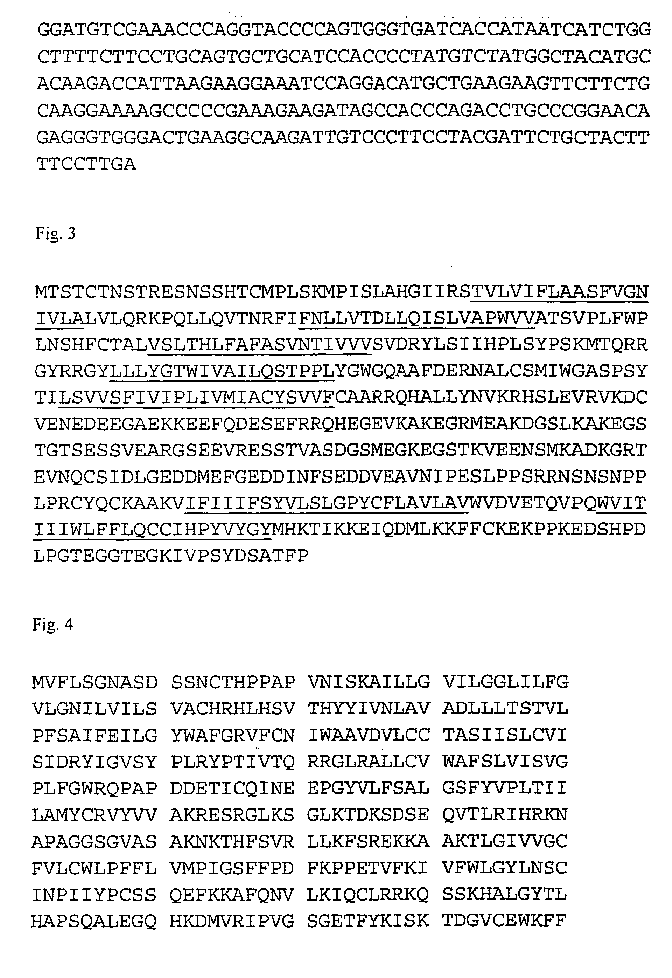

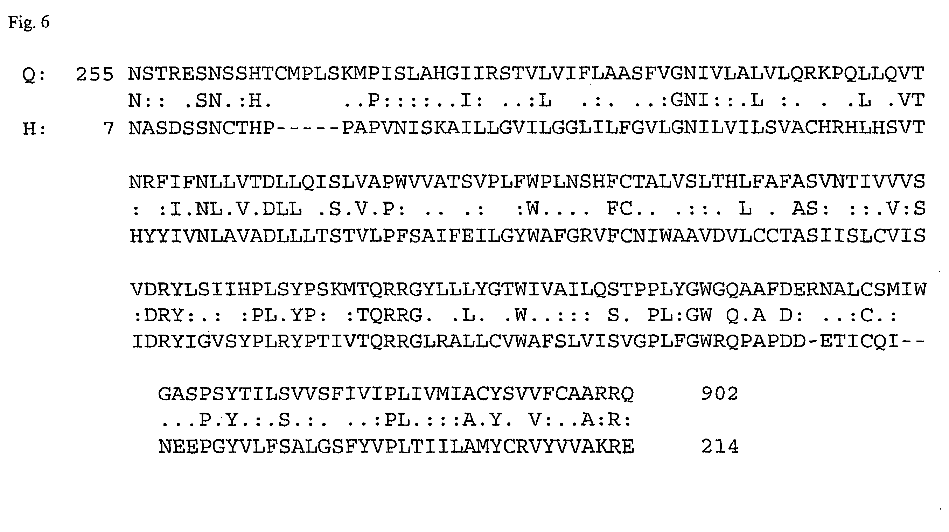

- Fig. 6: shows the alignment of α₁ₐ adrenergic receptor-like GPCR of Fig. 3 with SwissProt Accession No. O02824 of Fig. 4 (expectation value 4e-25).

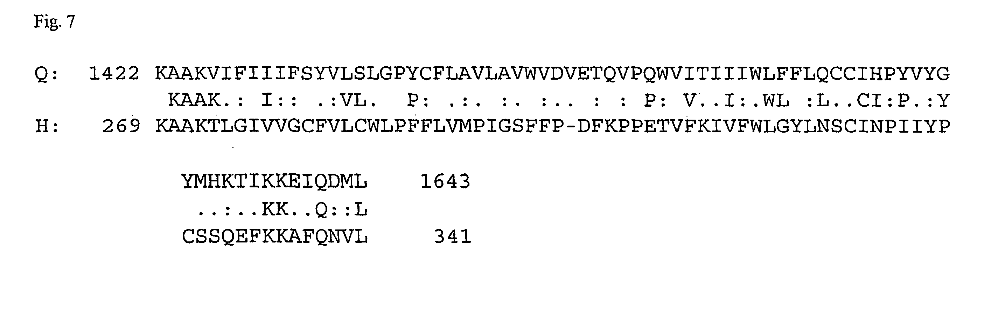

- Fig. 7: shows the BLASTX - alignment of α₁ₐ adrenergic receptor-like GPCR of Fig. 3 with SwissProt Accession No. 002824 of Fig. 4 (expectation value 0.013).

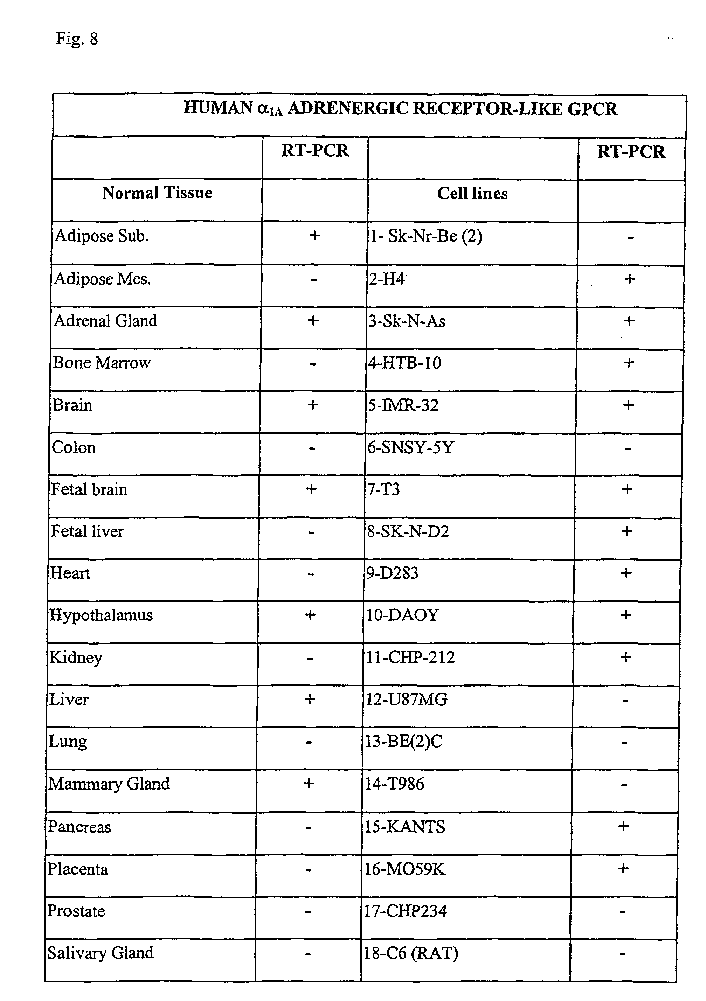

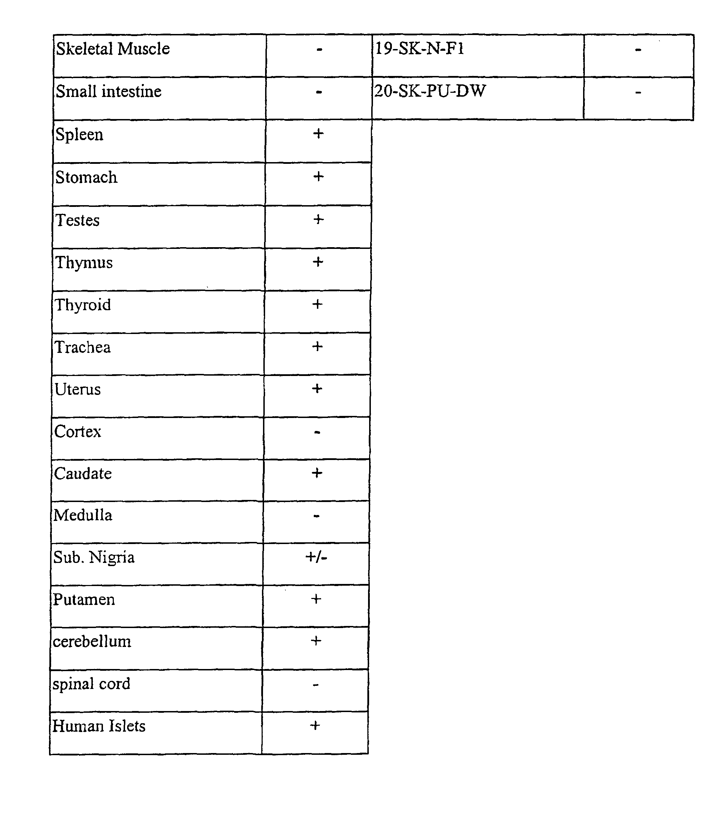

- Fig. 8: shows the relative expression of the α₁ₐ adrenergic receptor-like GPCR in different human tissues and cell lines as determined by RT-PCR.

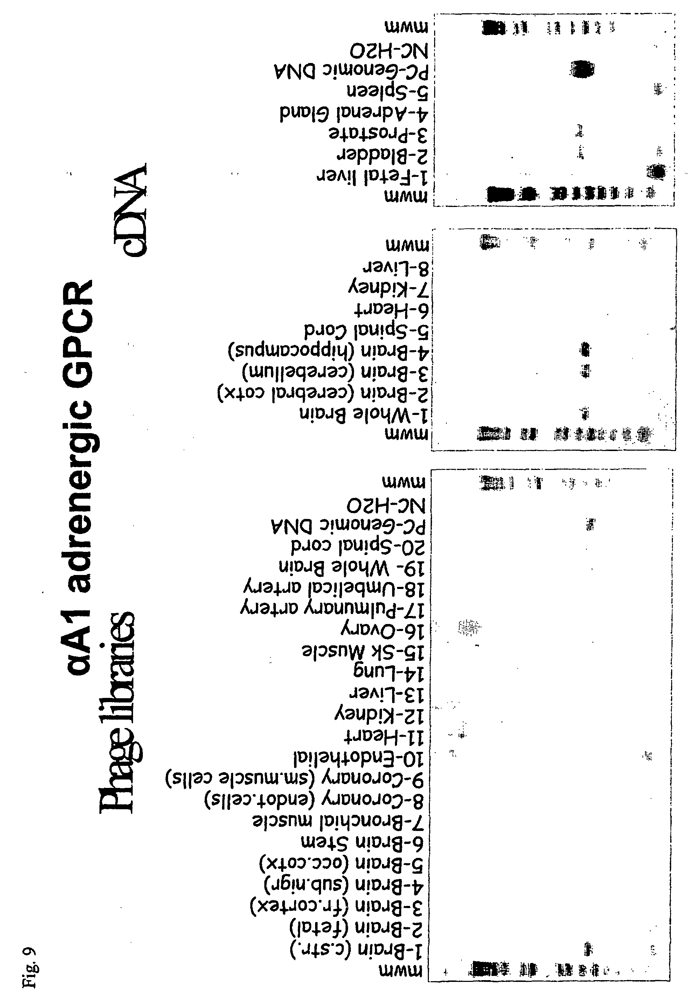

- Fig. 9: shows the relative expression of the α₁ₐ adrenergic receptor-like GPCR in different human tissues as determined by RT-PCR using phage libraries or cDNA as template

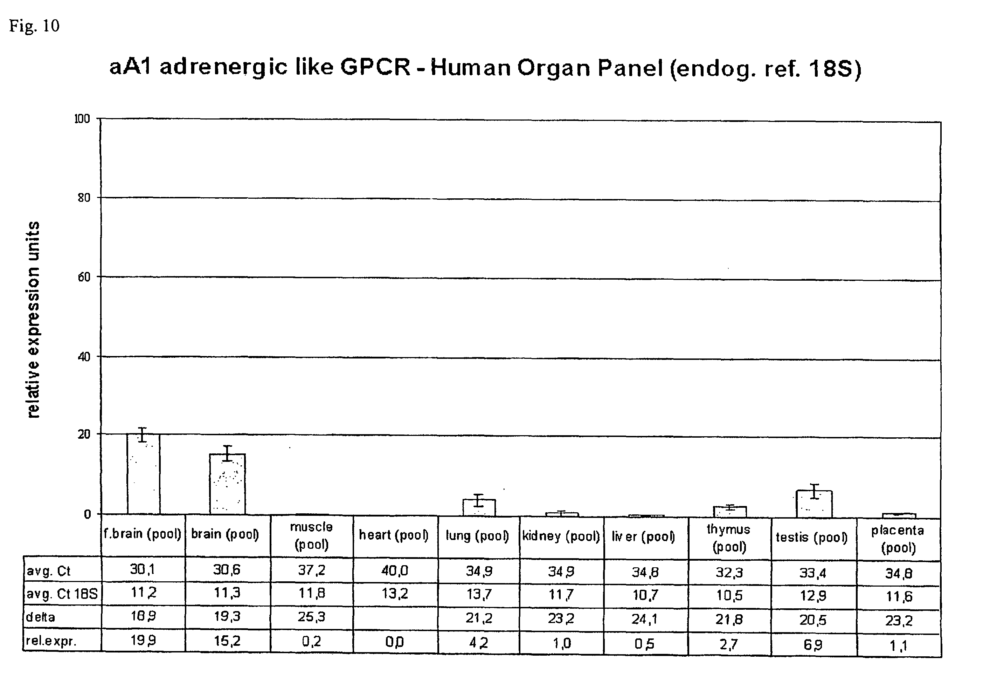

- Fig. 10: shows the relative expression of the α₁ₐ adrenergic receptor-like GPCR in different human tissues as determined by Taqman analysis

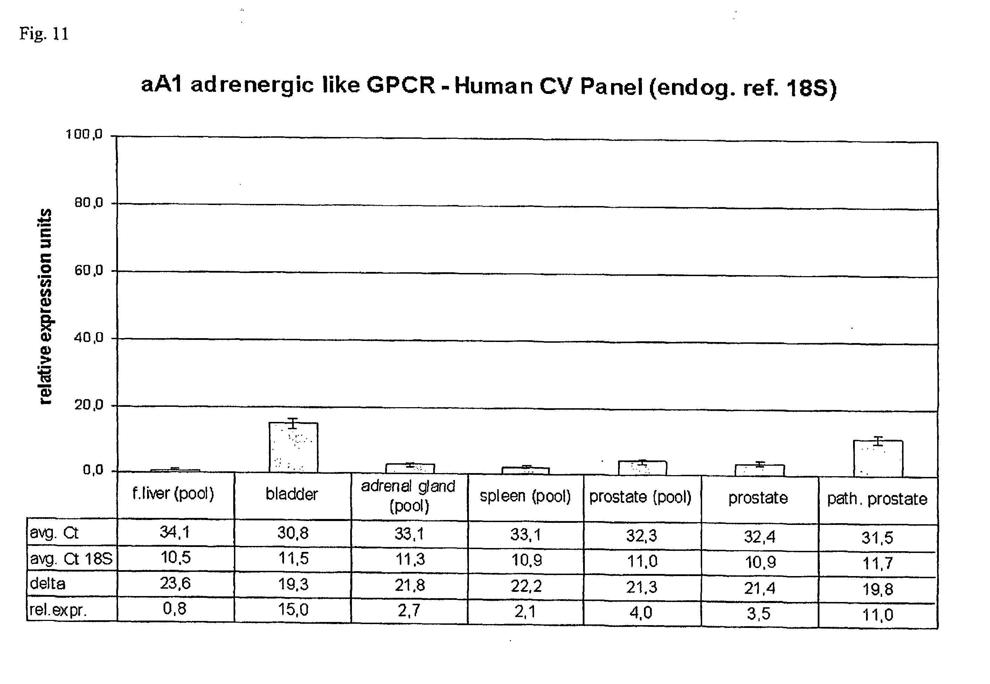

- Fig. 11: shows the relative expression of the α₁ₐ adrenergic receptor-like GPCR in different human tissues relevant for cardiovascular diseases as determined by Taqman analysis

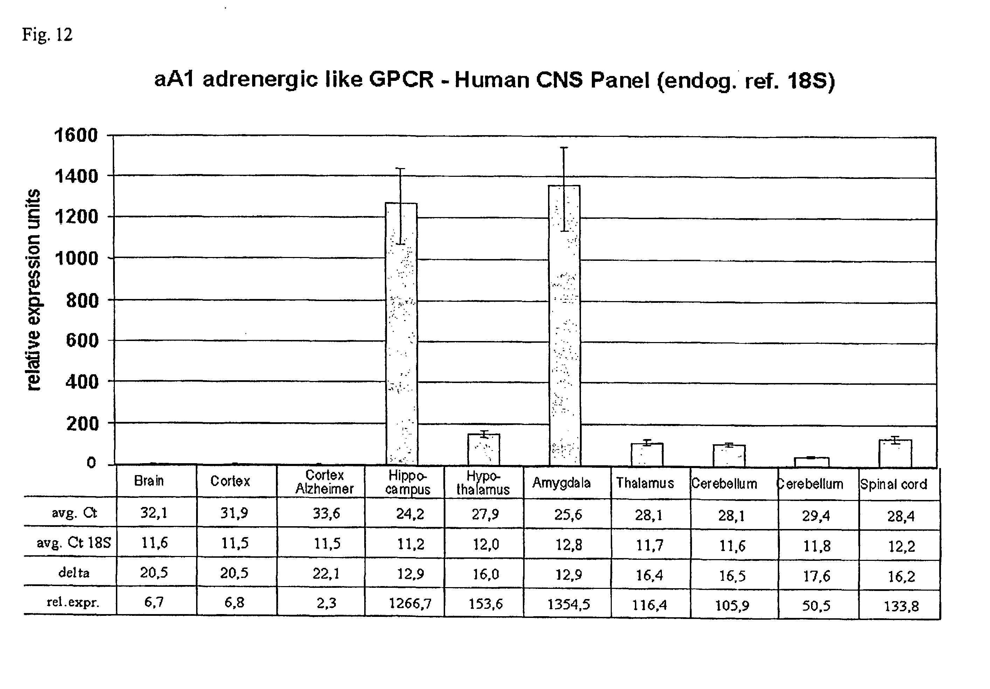

- Fig. 12: shows the relative expression of the α₁ₐ adrenergic receptor-like GPCR in different human tissues relevant for peripheral or central nervous system diseases as determined by Taqman analysis

### DETAILED DESCRIPTION OF THE INVENTION

The invention relates to an isolated polynucleotide encoding a α₁ₐ adrenergic receptor-like GPCR polypeptide.

Furthermore, it has been discovered by the present applicant that a α₁ₐ adrenergic receptor-like GPCR, particularly a human α₁ₐ adrenergic receptor-like GPCR, can be used in therapeutic methods to treat disorders such as pain, Parkinson's diseases, and psychotic and neurological disorders, including anxiety, schizophrenia, manic depression, delirium, dementia, several mental retardation, and dyskinesias, such as Huntington's disease and Tourett's syndrome. Human α1a adrenergic receptor-like GPCR also can be used to screen for α₁ₐ adrenergic receptor- like GPCR agonists and antagonists.

Human α₁ₐ adrenergic receptor-like GPCR is 30% identical over 216 amino acids to the *Oryctolagus cuniculus* protein having SwissProt Accession No. O02824 (SEQ ID NO.4) and annotated as an α₁ₐ adrenergic receptor (Fig. 4). Human α₁ₐ adrenergic receptor-like GPCR also is 29% identical over 74 amino acids to O02824. Human α₁ₐ adrenergic receptor-like GPCR therefore is expected to have an α₁ₐ adrenergic receptor- like function and to be useful for the same purposes as previously identified α₁ₐ adrenergic receptors. Human α₁ₐ adrenergic receptor-like GPCR has transmembrane regions from amino acids 36-53, 71-89, 108-125, 144-161, 189-210, 395-417, and 428-450.

### Biologically Active Variants

GPCR polypeptide variants which are biologically active, *i*.*e*., retain the ability to bind a ligand to produce a biological effect, such as cyclic AMP formation, mobilization of intracellular calcium, or phosphoinositide metabolism, also are GPCR polypeptides. Preferably, naturally or non-naturally occurring GPCR polypeptide variants have amino acid sequences which are at least about 50, preferably about 90% identical to an amino acid sequence shown in SEQ ID NO. 3 or a fragment thereof. Percent identity between a putative GPCR polypeptide variant and an amino acid sequence of SEQ ID NO. 3 is determined using the Blast2 alignment program (using Blosum62, Expect 10, standard genetic codes).

Variations in percent identity can be due, for example, to amino acid substitutions, insertions, or deletions. Amino acid substitutions are defined as one for one amino acid replacements. They are conservative in nature when the substituted amino acid has similar structural and/or chemical properties. Examples of conservative replacements are substitution of a leucine with an isoleucine or valine, an aspartate with a glutamate, or a threonine with a serine.

Amino acid insertions or deletions are changes to or within an amino acid sequence. They typically fall in the range of about 1 to 5 amino acids. Guidance in determining which amino acid residues can be substituted, inserted, or deleted without abolishing biological or immunological activity of a GPCR polypeptide can be found using computer programs well known in the art, such as DNASTAR software. Whether an amino acid change results in a biologically active GPCR polypeptide can readily be determined by assaying for binding to a ligand or by conducting a functional assay, as described for example, in the specific Examples, below.

### Fusion Proteins

Fusion proteins are useful for generating antibodies against GPCR polypeptide amino acid sequences and for use in various assay systems. For example, fusion proteins can be used to identify proteins which interact with portions of a GPCR polypeptide. Protein affinity chromatography or library-based assays for protein-protein interactions, such as the yeast two-hybrid or phage display systems, can be used for this purpose. Such methods are well known in the art and also can be used as drug screens.

A human α₁ₐ adrenergic receptor-like GPCR fusion protein comprises two polypeptide segments fused together by means of a peptide bond. The first polypeptide segment comprises at least 10, 20, 30, 40, 50, 75, 100, 125, 150, 175, 200, 250, 300, 350, 400, 450, or 500 contiguous amino acids of SEQ ID NO. 3 or a biologically active variant thereof, such as those described above. The first polypeptide segment also can comprise full-length human α₁ₐ adrenergic receptor-like GPCR.

The second polypeptide segment can be a full-length protein or a protein fragment. Proteins commonly used in fusion protein construction include β-galactosidase, β-glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions, including histidine (His) tags, FLAG tags, influenza hemagglutinin (HA) tags, Myc tags, VSV-G tags, and thioredoxin (Trx) tags. Other fusion constructions can include maltose binding protein (MBP), S-tag, Lex a DNA binding domain (DBD) fusions, GAL4 DNA binding domain fusions, and herpes simplex virus (HSV) BP16 protein fusions. A fusion protein also can be engineered to contain a cleavage site located between the GPCR polypeptide-encoding sequence and the heterologous protein sequence, so that the GPCR polypeptide can be cleaved and purified away from the heterologous moiety.

A fusion protein can be synthesized chemically, as is known in the art. Preferably, a fusion protein is produced by covalently linking two polypeptide segments or by standard procedures in the art of molecular biology. Recombinant DNA methods can be used to prepare fusion proteins, for example, by making a DNA construct which comprises coding sequences selected from the complement of SEQ ID NO. 2 in proper reading frame with nucleotides encoding the second polypeptide segment and expressing the DNA construct in a host cell, as is known in the art. Many kits for constructing fusion proteins are available from companies such as Promega Corporation (Madison, WI), Stratagene (La Jolla, CA), CLONTECH (Mountain View, CA), Santa Cruz Biotechnology (Santa Cruz, CA), MBL International Corporation (MIC; Watertown, MA), and Quantum Biotechnologies (Montreal, Canada; 1-888-DNA-KITS).

### Identification of Species Homologs

Species homologs of human α₁ₐ adrenergic receptor-like GPCR polypeptides can be obtained using polynucleotides of the invention (described below) to make suitable probes or primers for screening cDNA expression libraries from other species, such as mice, monkeys, or yeast, identifying cDNAs which encode homologs of GPCR polypeptide, and expressing the cDNAs as is known in the art.

### Polynucleotides

A human α₁ₐ adrenergic receptor-like GPCR polynucleotide can be single- or double-stranded and comprises a coding sequence or the complement of a coding sequence for a human α₁ₐ adrenergic receptor-like GPCR polypeptide. A coding sequence for human α₁ₐ adrenergic receptor-like GPCR is shown in SEQ ID NO. 2. The coding sequence obeys the classical KOZAK rule for the transcription start site. The coding sequence of SEQ ID NO.1 comprises this coding sequence and also contains a promoter module (EBOX_E2FF_01), which consists of two trans-acting binding domains, EBOX and E2F. The module is involved in the activation of the Epstein-Barr virus pol promoter. See Liu *et al*., *J. Virol. 70,* 2545-55, 1996. SEQ ID NO.1 also contains a promoter module (GATA_AP1F_01), which consists of two transacting binding domains, GATA and AP1F. The module is required for the constitutive expression of the IL-5 gene in adult T-cell leukemia cells. See Yamagata *et al*., *Mol. Cell. Biol. 17*, 4272-81, 1997.

Degenerate nucleotide sequences encoding human α₁ₐ adrenergic receptor-like GPCR polypeptides, as well as homologous nucleotide sequences which are at least about 50, preferably about 75, 90, 96, or 98% identical to the nucleotide sequences shown in SEQ ID NO.1 or 3 also are human α₁ₐ adrenergic receptor-like GPCR polynucleotides. Percent sequence identity between the sequences of two polynucleotides is determined using computer programs such as ALIGN which employ the PASTA algorithm, using an affine gap search with a gap open penalty of -12 and a gap extension penalty of -2. Complementary DNA (cDNA) molecules, species homologs, and variants of GPCR polynucleotides which encode biologically active GPCR polypeptides also are GPCR polynucleotides.

### Preparation of Polynucleotides

A naturally occurring polynucleotide can be isolated free of other cellular components such as membrane components, proteins, and lipids. Polynucleotides can be made by a cell and isolated using standard nucleic acid purification techniques, or synthesized using an amplification technique, such as the polymerase chain reaction (PCR), or by using an automatic synthesizer. Methods for isolating polynucleotides are routine and are known in the art. Any such technique for obtaining a polynucleotide can be used to obtain isolated polynucleotides. For example, restriction enzymes and probes can be used to isolate polynucleotide fragments which comprises human α₁ₐ adrenergic receptor- like GPCR nucleotide sequences. Isolated polynucleotides are in preparations which are free or at least 70, 80, or 90% free of other molecules.

Human α₁ₐ adrenergic receptor-like GPCR cDNA molecules can be made with standard molecular biology techniques, using human α₁ₐ adrenergic receptor-like GPCR mRNA as a template. cDNA molecules can thereafter be replicated using molecular biology techniques known in the art and disclosed in manuals such as Sambrook *et al*. (1989). An amplification technique, such as PCR, can be used to obtain additional copies of polynucleotides of the invention, using either human genomic DNA or cDNA as a template.

Alternatively, synthetic chemistry techniques can be used to synthesize human α₁ₐ adrenergic receptor-like GPCR polynucleotides. The degeneracy of the genetic code allows alternate nucleotide sequences to be synthesized which will encode a polypeptide having, for example, an amino acid sequence shown in SEQ ID NO. 3 or a biologically active variant thereof.

### Extending Polynucleotides

Various PCR-based methods can be used to extend the nucleic acid sequences encoding the disclosed portions of human α₁ₐ adrenergic receptor-like GPCR to detect upstream sequences such as promoters and regulatory elements. For example, restriction-site PCR uses universal primers to retrieve unknown sequence adjacent to a known locus (Sarkar, *PCR Methods Applic. 2*, 318-322, 1993). Genomic DNA is first amplified in the presence of a primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Inverse PCR also can be used to amplify or extend sequences using divergent primers based on a known region (Triglia *et al*., *Nucleic Acids Res. 16,* 8186, 1988). Primers can be designed using commercially available software, such as OLIGO 4.06 Primer Analysis software (National Biosciences Inc., Plymouth, Minn.), to be 22-30 nucleotides in length, to have a GC content of 50% or more, and to anneal to the target sequence at temperatures about 68-72°C. The method uses several restriction enzymes to generate a suitable fragment in the known region of a gene. The fragment is then circularized by intramolecular ligation and used as a PCR template.

Another method which can be used is capture PCR, which involves PCR amplification of DNA fragments adjacent to a known sequence in human and yeast artificial chromosome DNA (Lagerstrom *et al*., *PCR Methods Applic. 1*, 111-119, 1991). In this method, multiple restriction enzyme digestions and ligations also can be used to place an engineered double-stranded sequence into an unknown fragment of the DNA molecule before performing PCR.

Another method which can be used to retrieve unknown sequences is that of Parker *et al*., *Nucleic Acids Res. 19,* 3055-3060, 1991). Additionally, PCR, nested primers, and PROMOTERFINDER libraries (CLONTECH, Palo Alto, Calif.) can be used to walk genomic DNA (CLONTECH, Palo Alto, Calif.). This process avoids the need to screen libraries and is useful in finding intron/exon junctions.

When screening for full-length cDNAs, it is preferable to use libraries that have been size-selected to include larger cDNAs. Randomly-primed libraries are preferable, in that they will contain more sequences which contain the 5' regions of genes. Use of a randomly primed library may be especially preferable for situations in which an oligo d(T) library does not yield a full-length cDNA. Genomic libraries can be useful for extension of sequence into 5' non-transcribed regulatory regions.

Commercially available capillary electrophoresis systems can be used to analyze the size or confirm the nucleotide sequence of PCR or sequencing products. For example, capillary sequencing can employ flowable polymers for electrophoretic separation, four different fluorescent dyes (one for each nucleotide) which are laser activated, and detection of the emitted wavelengths by a charge coupled device camera. Output/light intensity can be converted to electrical signal using appropriate software (*e*.*g*. GENOTYPER and Sequence NAVIGATOR, Perkin Elmer), and the entire process from loading of samples to computer analysis and electronic data display can be computer controlled. Capillary electrophoresis is especially preferable for the sequencing of small pieces of DNA which might be present in limited amounts in a particular sample.

### Obtaining GPCR Polypeptides

Human α₁ₐ adrenergic receptor-like GPCR polypeptides can be obtained, for example, by purification from human cells, by expression of polynucleotides, or by direct chemical synthesis.

### Protein Purification

Human α₁ₐ adrenergic receptor-like GPCR polypeptides can be purified from any human cell which expresses the receptor, including host cells which have been transfected with human α₁ₐ adrenergic receptor-like GPCR polynucleotides. A purified polypeptide is separated from other compounds which normally associate with the polypeptide in the cell, such as certain proteins, carbohydrates, or lipids, using methods well-known in the art Such methods include, but are not limited to, size exclusion chromatography, ammonium sulfate fractionation, ion exchange chromatography, affinity chromatography, and preparative gel electrophoresis.

A human α₁ₐ adrenergic receptor-like GPCR polypeptide can be conveniently isolated as a complex with its associated G protein, as described in the specific examples, below. A preparation of purified human α₁ₐ adrenergic receptor-like GPCR polypeptides is at least 80% pure; preferably, the preparations are 90%, 95%, or 99% pure. Purity of the preparations can be assessed by any means known in the art, such as SDS-polyacrylamide gel electrophoresis.

### Expression of Polynucleotides

To express a human α₁ₐ adrenergic receptor-like GPCR polypeptide, a human α₁ₐ adrenergic receptor-like GPCR polynucleotide can be inserted into an expression vector which contains the necessary elements for the transcription and translation of the inserted coding sequence. Methods which are well known to those skilled in the art can be used to construct expression vectors containing sequences encoding human α₁ₐ adrenergic receptor- like GPCR polypeptides and appropriate transcriptional and translational control elements. These methods include *in vitro* recombinant DNA techniques, synthetic techniques, and *in vivo* genetic recombination. Such techniques are described, for example, in Sambrook *et al*. (1989) and in Ausubel *et al*., CURRENT PROTOCOLS IN MOLECULAR BIOLOGY, John Wiley & Sons, New York, N.Y., 1989.

A variety of expression vector/host systems can be utilized to contain and express sequences encoding a human α₁ₐ adrenergic receptor-like GPCR polypeptide. These include, but are not limited to, microorganisms, such as bacteria transformed with recombinant bacteriophage, plasmid, or cosmid DNA expression vectors; yeast transformed with yeast expression vectors, insect cell systems infected with virus expression vectors (*e*.*g*., baculovirus), plant cell systems transformed with virus expression vectors (*e*.*g*., cauliflower mosaic virus, CaMV; tobacco mosaic virus, TMV) or with bacterial expression vectors (*e*.*g*., Ti or pBR322 plasmids), or animal cell systems.

The control elements or regulatory sequences are those non-translated regions of the vector - enhancers, promoters, 5' and 3' untranslated regions - which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORT1 plasmid (Life Technologies) and the like can be used. The baculovirus polyhedrin promoter can be used in insect cells. Promoters or enhancers derived from the genomes of plant cells *(e.g.,* heat shock, RUBISCO, and storage protein genes) or from plant viruses (*e*.*g*., viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding a GPCR polypeptide, vectors based on SV40 or EBV can be used with an appropriate selectable marker.

### Bacterial and Yeast Expression Systems

In bacterial systems, a number of expression vectors can be selected depending upon the use intended for the GPCR polypeptide. For example, when a large quantity of a GPCR polypeptide is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding the GPCR polypeptide can be ligated into the vector in frame with sequences for the amino-terminal Met and the subsequent 7 residues of β-galactosidase so that a hybrid protein is produced. pIN vectors (Van Heeke & Schuster, *J. Biol. Chem*. *264*, 5503-5509, 1989) or pGEX vectors (Promega, Madison, Wis.) also can be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems can be designed to include heparin, thrombin, or factor Xa protease cleavage sites so that the cloned polypeptide of interest can be released from the GST moiety at will.

In the yeast *Saccharomyces cerevisiae,* a number of vectors containing constitutive or inducible promoters such as alpha factor, alcohol oxidase, and PGH can be used. For reviews, see Ausubel *et al*. (1989) and Grant *et al*., *Methods Enzymol. 153, 516*-544, 1987.

### Plant and Insect Expression Systems

If plant expression vectors are used, the expression of sequences encoding GPCR polypeptides can be driven by any of a number of promoters. For example, viral promoters such as the 35S and 19S promoters of CaMV can be used alone or in combination with the omega leader sequence from TMV (Takamatsu, *EMBO J. 6,* 307-311, 1987). Alternatively, plant promoters such as the small subunit of RUBISCO or heat shock promoters can be used (Coruzzi *et al*., *EMBO J. 3*, 1671-1680, 1984; Broglie *et al., Science 224, 838-843,* 1984; Winter *et al*., *Results Probl. Cell Differ. 17,* 85-105, 1991). These constructs can be introduced into plant cells by direct DNA transformation or by pathogen-mediated transfection. Such techniques are described in a number of generally available reviews (*e*.*g*., Hobbs or Murray, in MCGRAW HELL YEARBOOK OF SCIENCE AND TECHNOLOGY, McGraw Hill, New York, N.Y., pp. 191-196, 1992).

An insect system also can be used to express a GPCR polypeptide. For example, in one such system *Autographa californica* nuclear polyhedrosis virus (AcNPV) is used as a vector to express foreign genes in *Spodoptera frugiperda* cells or in *Trichoplusia* larvae. Sequences encoding GPCR polypeptides can be cloned into a non-essential region of the virus, such as the polyhedrin gene, and placed under control of the polyhedrin promoter. Successful insertion of GPCR polypeptides will render the polyhedrin gene inactive and produce recombinant virus lacking coat protein. The recombinant viruses can then be used to infect *S*. *frugiperda cells or Trichoplusia* larvae in which GPCR polypeptides can be expressed (Engelhard *et al., Proc. Nat. Acad. Sci. 91*, 3224-3227, 1994).

### Mammalian Expression Systems

A number of viral-based expression systems can be used to express GPCR polypeptides in mammalian host cells. For example, if an adenovirus is used as an expression vector, sequences encoding GPCR polypeptides can be ligated into an adenovirus transcription/translation complex comprising the late promoter and tripartite leader sequence. Insertion in a non-essential E1 or E3 region of the viral genome can be used to obtain a viable virus which is capable of expressing a GPCR polypeptide in infected host cells (Logan & Shenk, *Proc. Natl. Acad. Sci. 81*, 3655-3659, 1984). If desired, transcription enhancers, such as the Rous sarcoma virus (RSV) enhancer, can be used to increase expression in mammalian host cells.

Human artificial chromosomes (HACs) also can be used to deliver larger fragments of DNA than can be contained and expressed in a plasmid. HACs of 6M to 10M are constructed and delivered to cells via conventional delivery methods (*e*.*g*., liposomes, polycationic amino polymers, or vesicles).

Specific initiation signals also can be used to achieve more efficient translation of sequences encoding GPCR polypeptides. Such signals include the ATG initiation codon and adjacent sequences. In cases where sequences encoding a GPCR polypeptide, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic. The efficiency of expression can be enhanced by the inclusion of enhancers which are appropriate for the particular cell system which is used (see Scharf *et al., Results Probl. Cell Differ. 20*, 125-162, 1994).

### Host Cells

A host cell strain can be chosen for its ability to modulate the expression of the inserted sequences or to process the expressed GPCR polypeptide in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for post-translational activities (*e*.*g*., CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express GPCR polypeptides can be transformed using expression vectors which can contain viral origins of replication and/or endogenous expression elements and a selectable marker gene on the same or on a separate vector. Following the introduction of the vector, cells can be allowed to grow for 1-2 days in an enriched medium before they are switched to a selective medium. The purpose of the selectable marker is to confer resistance to selection, and its presence allows growth and recovery of cells which successfully express the introduced GPCR sequences. Resistant clones of stably transformed cells can be proliferated using tissue culture techniques appropriate to the cell type. See, for example, ANIMAL CELL CULTURE, R.I. Freshney, ed., 1986.

Any number of selection systems can be used to recover transformed cell lines.

These include, but are not limited to, the herpes simplex virus thymidine kinase (Wigler *et al., Cell 11*, 223-32, 1977) and adenine phosphoribosyltransferase (Lowy *et al*., *Cell 22,* 817-23, 1980) genes which can be employed in *tk*^{*-*} or *aprt*^{*-*} cells, respectively. Also, antimetabolite, antibiotic, or herbicide resistance can be used as the basis for selection. For example, *dhfr* confers resistance to methotrexate (Wigler *et al*., *Proc. Natl. Acad. Sci. 77*, 3567-70, 1980), *npt* confers resistance to the aminoglycosides, neomycin and G-418 (Colbere-Garapin *et al*., *J. Mol. Biol. 150*, 1-14, 1981), and *als* and *pat* confer resistance to chlorsulfuron and phosphinotricin acetyltransferase, respectively (Murray, 1992, *supra).* Additional selectable genes have been described. For example, *trpB* allows cells to utilize indole in place of tryptophan, or *hisD,* which allows cells to utilize histinol in place of histidine (Hardman & Mulligan, *Proc. Natl. Acad Sci. 85*, 8047-51, 1988). Visible markers such as anthocyanins, β-glucuronidase and its substrate GUS, and luciferase and its substrate luciferin, can be used to identify transformants and to quantify the amount of transient or stable protein expression attributable to a specific vector system (Rhodes *et al., Methods Mol. Biol*. *55*,121-131, 1995).

### Detecting Expression of Polypeptides

Although the presence of marker gene expression suggests that the human α₁ₐ adrenergic receptor-like GPCR polynucleotide is also present, its presence and expression may need to be confirmed. For example, if a sequence encoding a human α₁ₐ adrenergic receptor-like GPCR polypeptide is inserted within a marker gene sequence, transformed cells containing sequences which encode a human α₁ₐ adrenergic receptor-like GPCR polypeptide can be identified by the absence of marker gene function. Alternatively, a marker gene can be placed in tandem with a sequence encoding a human α₁ₐ adrenergic receptor-like GPCR polypeptide under the control of a single promoter. Expression of the marker gene in response to induction or selection usually indicates expression of the GPCR polynucleotide.

Alternatively, host cells which contain a human α₁ₐ adrenergic receptor-like GPCR polynucleotide and which express a human α₁ₐ adrenergic receptor-like GPCR polypeptide can be identified by a variety of procedures known to those of skill in the art. These procedures include, but are not limited to, DNA-DNA or DNA-RNA hybridizations and protein bioassay or immunoassay techniques which include membrane, solution, or chip-based technologies for the detection and/or quantification of nucleic acid or protein. For example, the presence of a polynucleotide sequence encoding a human α₁ₐ adrenergic receptor-like GPCR polypeptide can be detected by DNA-DNA or DNA-RNA hybridization or amplification using probes or fragments or fragments of polynucleotides encoding a GPCR polypeptide. Nucleic acid amplification-based assays involve the use of oligonucleotides selected from sequences encoding a human α₁ₐ adrenergic receptor-like GPCR polypeptide to detect transformants which contain a human α₁ₐ adrenergic receptor-like GPCR polynucleotide.

A variety of protocols for detecting and measuring the expression of a human α₁ₐ adrenergic receptor-like GPCR polypeptide, using either polyclonal or monoclonal antibodies specific for the polypeptide, are known in the art. Examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on a human α₁ₐ adrenergic receptor-like GPCR polypeptide can be used, or a competitive binding assay can be employed. These and other assays are described in Hampton *et al*., SEROLOGICAL METHODS: A LABORATORY MANUAL, APS Press, St. Paul, Minn., 1990) and Maddox *et al*., *J. Exp. Med 158*, 1211-1216, 1983).

A wide variety of labels and conjugation techniques are known by those skilled in the art and can be used in various nucleic acid and amino acid assays. Means for producing labeled hybridization or PCR probes for detecting sequences related to polynucleotides encoding human α₁ₐ adrenergic receptor-like GPCR polypeptides include oligolabeling, nick translation, end-labeling, or PCR amplification using a labeled nucleotide. Alternatively, sequences encoding a human α₁ₐ adrenergic receptor-like GPCR polypeptide can be cloned into a vector for the production of an mRNA probe. Such vectors are known in the art, are commercially available, and can be used to synthesize RNA probes *in vitro* by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

### Expression and Purification of GPCR Polypeptides

Host cells transformed with nucleotide sequences encoding a human α₁ₐ adrenergic receptor-like GPCR polypeptide can be cultured under conditions suitable for the expression and recovery of the protein from cell culture. The polypeptide produced by a transformed cell can be secreted or contained intracellularly depending on the sequence and/or the vector used. As will be understood by those of skill in the art, expression vectors containing polynucleotides which encode human α₁ₐ adrenergic receptor-like GPCR polypeptides can be designed to contain signal sequences which direct secretion of soluble polypeptides through a prokaryotic or eukaryotic cell membrane or which direct the membrane insertion of membrane-bound polypeptide.

As discussed above, other constructions can be used to join a sequence encoding a human α₁ₐ adrenergic receptor-like GPCR polypeptide to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor Xa or enterokinase (Invitrogen, San Diego, CA) between the purification domain and the human α₁ₐ adrenergic receptor-like GPCR polypeptide also can be used to facilitate purification. One such expression vector provides for expression of a fusion protein containing a human α₁ₐ adrenergic receptor-like GPCR polypeptide and 6 histidine residues preceding a thioredoxin or an enterokinase cleavage site. The histidine residues facilitate purification by IMAC (immobilized metal ion affinity chromatography, as described in Porath *et al., Prot. Exp. Purif. 3*, 263-281, 1992), while the enterokinase cleavage site provides a means for purifying the polypeptide from the fusion protein. Vectors which contain fusion proteins are disclosed in Kroll *et al*., *DNA Cell Biol*. *12*, 441-453, 1993.

### Chemical Synthesis

Sequences encoding a human α₁ₐ adrenergic receptor-like GPCR polypeptide can be synthesized, in whole or in part, using chemical methods well known in the art (see Caruthers *et al*., *Nucl*. *Acids Res*. *Symp*. *Ser*. 215-223, 1980; Horn *et al*. *Nucl*. *Acids Res. Symp*. *Ser*. 225-232, 1980). Alternatively, a GPCR polypeptide itself can be produced using chemical methods to synthesize its amino acid sequence, such as by direct peptide synthesis using solid-phase techniques (Merrifield, *J. Am. Chem*. *Soc. 85*, 2149-2154, 1963; Roberge *et al*., *Science 269, 202-204,* 1995). Protein synthesis can be performed using manual techniques or by automation. Automated synthesis can be achieved, for example, using Applied Biosystems 431A Peptide Synthesizer (Perkin Elmer). Optionally, fragments of GPCR polypeptides can be separately synthesized and combined using chemical methods to produce a full-length molecule.

The newly synthesized peptide can be substantially purified by preparative high performance liquid chromatography (*e*.*g*., Creighton, PROTEINS: STRUCTURES AND MOLECULAR PRINCIPLES, WH Freeman and Co., New York, N.Y., 1983). The composition of a synthetic human α₁ₐ adrenergic recegtor-like GPCR polypeptide can be confirmed by amino acid analysis or sequencing (*e*.*g*., the Edman degradation procedure; *see* Creighton, *supra*). Additionally, any portion of the amino acid sequence of the human α₁ₐ adrenergic receptor-like GPCR polypeptide can be altered during direct synthesis and/or combined using chemical methods with sequences from other proteins to produce a variant polypeptide or a fusion protein.

### Screening Methods

The invention provides assays for screening test compounds which bind to or modulate the activity of a human α₁ₐ adrenergic receptor-like GPCR polypeptide or a polynucleotide. A test compound preferably binds to a human α₁ₐ adrenergic receptor-like GPCR polypeptide or polynucleotide. More preferably, a test compound decreases or increases a biological activity mediated via human α₁ₐ adrenergic receptor-like GPCR by at least about 10, preferably about 50, more preferably about 75,90, or 100% relative to the absence of the test compound.

### Test Compounds

Test compounds can be pharmacologic agents already known in the art or can be compounds previously unknown to have any pharmacological activity. The compounds can be naturally occurring or designed in the laboratory. They can be isolated from microorganisms, animals, or plants, and can be produced recombinantly, or synthesized by chemical methods known in the art. If desired, test compounds can be obtained using any of the numerous combinatorial library methods known in the art, including but not limited to, biological libraries, spatially addressable parallel solid phase or solution phase libraries, synthetic library methods requiring deconvolution, the "one-bead one-compound" library method, and synthetic library methods using affinity chromatography selection. The biological library approach is limited to polypeptide libraries, while the other four approaches are applicable to polypeptide, non-peptide oligomer, or small molecule libraries of compounds. *See* Lam, *Anticancer Drug Des. 12,145,1997.*

Methods for the synthesis of molecular libraries are well known in the art *(see,* for example, DeWitt *et al., Proc. Natl. Acad Sci. U.S.A. 90,* 6909, 1993; Erb *et al. Proc. Natl. Acad. Sci. USA. 91*, 11422, 1994; Zuckermann *et al., J. Med. Chem.* 37, 2678, 1994; Cho *et al., Science 261,* 1303, 1993; Carell *et al*., *Angew. Chem. Int. Ed Engl.* 33, 2059, 1994; Carell *et al., Angew. Chem. Int. Ed. Engl. 33, 2061;* Gallop *et al., J.* *Med. Chem. 37*, 1233, 1994). Libraries of compounds can be presented in solution (*see*, *e*.*g*., Houghten, *BioTechniques 13*, 412-421, 1992), or on beads (Lam, *Nature 354,* 82-84, 1991), chips (Fodor, *Nature 364,* 555-556, 1993), bacteria or spores (Ladner, U.S. Patent 5,223,409), plasmids (Cull *et al*., *Proc. Natl. Acad Sci. U.S.A. 89*, 1865-1869, 1992), or phage (Scott & Smith, *Science 249*, 386-390, 1990; Devlin, *Science 249*, 404-406, 1990); Gwirla *et al., Proc. Natl. Acad Sci. 97,* 6378-6382, 1990; Felici, *J. Mol. Biol. 222*, 301-310, 1991; and Ladner, U.S. Patent 5,223,409).

### High Throughput Screening

Test compounds can be screened for the ability to bind to human α₁ₐ adrenergic receptor-like GPCR polypeptides or polynucleotides or to affect human α₁ₐ adrenergic receptor-like GPCR activity or gene expression using high throughput screening. Using high throughput screening, many discrete compounds can be tested in parallel so that large numbers of test compounds can be quickly screened. The most widely established techniques utilize 96-well microtiter plates. The wells of the microtiter plates typically require assay volumes that range from 50 to 500 µl. In addition to the plates, many instruments, materials, pipettors, robotics, plate washers, and plate readers are commercially available to fit the 96-well format.

Alternatively, "free format assays," or assays that have no physical barrier between samples, can be used. For example, an assay using pigment cells (melanocytes) in a simple homogeneous assay for combinatorial peptide libraries is described by Jayawickreme *et al*., *Proc. Natl. Acad Sci. U.S.A. 19*, 1614-18 (1994). The cells are placed under agarose in petri dishes, then beads that carry combinatorial compounds are placed on the surface of the agarose. The combinatorial compounds are partially released the compounds from the beads. Active compounds can be visualized as dark pigment areas because, as the compounds diffuse locally into the gel matrix, the active compounds cause the cells to change colors.

Another example of a free format assay is described by Chelsky, "Strategies for Screening Combinatorial Libraries: Novel and Traditional Approaches," reported at the First Annual Conference of The Society for Biomolecular Screening in Philadelphia, Pa. (Nov. 7-10, 1995). Chelsky placed a simple homogenous enzyme assay for carbonic anhydrase inside an agarose gel such that the enzyme in the gel would cause a color change throughout the geL Thereafter, beads carrying combinatorial compounds via a photolinker were placed inside the gel and the compounds were partially released by UV-light. Compounds that inhibited the enzyme were observed as local zones of inhibition having less color change.

Yet another example is described by Salmon *et al*., *Molecular Diversity 2*, 57-63 (1996). In this example, combinatorial libraries were screened for compounds that had cytotoxic effects on cancer cells growing in agar.

Another high throughput screening method is described in Beutel *et al*., U.S. Patent 5,976,813. In this method, test samples are placed in a porous matrix. One or more assay components are then placed within, on top of, or at the bottom of a matrix such as a gel, a plastic sheet, a filter, or other form of easily manipulated solid support. When samples are introduced to the porous matrix they diffuse sufficiently slowly, such that the assays can be performed without the test samples running together.

### Binding Assays

For binding assays, the test compound is preferably a small molecule which binds to and occupies the ligand binding site of the human α₁ₐ adrenergic receptor-like GPCR polypeptide, thereby making the ligand binding site inaccessible to substrate such that normal biological activity is prevented. Examples of such small molecules include, but are not limited to, small peptides or peptide-like molecules. Potential ligands which bind to a polypeptide of the invention include, but are not limited to, the natural ligands of known GPCRs and analogues or derivatives thereof. Natural ligands of GPCRs include adrenomedullin, amylin, calcitonin gene related protein (CGRP), calcitonin, anandamide, serotonin, histamine, norepinephrine, adrenalin, noradrenalin, platelet activating factor, thrombin, C5a, bradykinin, and chemokines.

In binding assays, either the test compound or the human α₁ₐ adrenergic receptor-like GPCR polypeptide can comprise a detectable label, such as a fluorescent, radioisotopic, chemiluminescent, or enzymatic label, such as horseradish peroxidase, alkaline phosphatase, or luciferase. Detection of a test compound which is bound to the human α₁ₐ adrenergic receptor-like GPCR polypeptide can then be accomplished, for example, by direct counting of radioemmission, by scintillation counting, or by determining conversion of an appropriate substrate to a detectable product.

Alternatively, binding of a test compound to a human α₁ₐ adrenergic receptor-like GPCR polypeptide can be determined without labeling either of the interactants. For example, a microphysiometer can be used to detect binding of a test compound with a human α₁ₐ adrenergic receptor-like GPCR polypeptide. A microphysiometer (*e*.*g*., Cytosensor™) is an analytical instrument that measures the rate at which a cell acidifies its environment using a light-addressable potentiometric sensor (LAPS). Changes in this acidification rate can be used as an indicator of the interaction between a test compound and a human α₁ₐ adrenergic receptor-like GPCR polypeptide (McConnell *et al., Science* 257, 1906-1912, 1992).

Determining the ability of a test compound to bind to a human α₁ₐ adrenergic receptor-like GPCR polypeptide also can be accomplished using a technology such as real-time Bimolecular Interaction Analysis (BIA) (Sjolander & Urbaniczky, *Anal*. *Chem. 63*, 2338-2345, 1991, and Szabo *et al., Curr. Opin*. *Struct. Biol. 5*, 699-705, 1995). BIA is a technology for studying bzospecific interactions in real time, without labeling any of the interactants (*e*.*g*., BIAcore™). Changes in the optical phenomenon surface plasmon resonance (SPR) can be used as an indication of real-time reactions between biological molecules.

In yet another aspect of the invention, a human α₁ₐ adrenergic receptor-like GPCR polypeptide can be used as a "bait protein" in a two-hybrid assay or three-hybrid assay (see, *e*.*g*., U.S. Patent 5,283,317; Zervos *et al*., *Cell 72,* 223-232, 1993; Madura *et al*., *J. Biol. Chem. 268,* 12046-12054, 1993; Bartel *et al*., *BioTechniques 14*, 920-924, 1993; Iwabuchi *et al*., *Oncogene 8,* 1693-1696, 1993; and Brent W094/10300), to identify other proteins which bind to or interact with the human α₁ₐ adrenergic receptor-like GPCR polypeptide and modulate its activity.

The two-hybrid system is based on the modular nature of most transcription factors, which consist of separable DNA-binding and activation domains. Briefly, the assay utilizes two different DNA constructs. For example, in one construct, polynucleotide encoding a human α₁ₐ adrenergic receptor-like GPCR polypeptide can be fused to a polynucleotide encoding the DNA binding domain of a known transcription factor (*e*.*g*., GAL-4). In the other construct a DNA sequence that encodes an unidentified protein ("prey" or "sample") can be fused to a polynucleotide that codes for the activation domain of the known transcription factor. If the "bait" and the "prey" proteins are able to interact *in vivo* to form an protein-dependent complex, the DNA-binding and activation domains of the transcription factor are brought into close proximity. This proximity allows transcription of a reporter gene (*e*.*g*., LacZ), which is operably linked to a transcriptional regulatory site responsive to the transcription factor. Expression of the reporter gene can be detected, and cell colonies containing the functional transcription factor can be isolated and used to obtain the DNA sequence encoding the protein which interacts with the human α₁ₐ adrenergic receptor-like GPCR polypeptide.

It may be desirable to immobilize either the human α₁ₐ adrenergic receptor-like GPCR polypeptide (or polynucleotide) or the test compound to facilitate separation of bound from unbound forms of one or both of the interactants, as well as to accommodate automation of the assay. Thus, either the human α₁ₐ adrenergic receptor-like GPCR polypeptide (or polynucleotide) or the test compound can be bound to a solid support. Suitable solid supports include, but are not limited to, glass or plastic slides, tissue culture plates, microtiter wells, tubes, silicon chips, or particles such as beads (including, but not-limited to, latex, polystyrene, or glass beads). Any method known in the art can be used to attach the human α₁ₐ adrenergic receptor-like GPCR polypeptide (or polynucleotide) or test compound to a solid support, including use of covalent and non-covalent linkages, passive absorption, or pairs of binding moieties attached respectively to the polypeptide (or polynucleotide) or test compound and the solid support. Test compounds are preferably bound to the solid support in an array, so that the location of individual test compounds can be tracked. Binding of a test compound to a human α₁ₐ adrenergic receptor-like GPCR polypeptide (or polynucleotide) can be accomplished in any vessel suitable for containing the reactants. Examples of such vessels include microtiter plates, test tubes, and microcentrifuge tubes.

In one embodiment, the human α₁ₐ adrenergic receptor-like GPCR polypeptide is a fusion protein comprising a domain that allows the polypeptide to be bound to a solid support. For example, glutathione-S-transferase fusion proteins can be adsorbed onto glutathione sepharose beads (Sigma Chemical, St. Louis, Mo.) or glutathione derivatized microtiter plates, which are then combined with the test compound or the test compound and the non-adsorbed human α₁ₐ adrenergic receptor-like GPCR polypeptide; the mixture is then incubated under conditions conducive to complex formation (*e*.*g*., at physiological conditions for salt and pH). Following incubation, the beads or microtiter plate wells are washed to remove any unbound components. Binding of the interactants can be determined either directly or indirectly, as described above. Alternatively, the complexes can be dissociated from the solid support before binding is determined.

Other techniques for immobilizing proteins or polynucleotides on a solid support also can be used in the screening assays of the invention. For example, either a human α₁ₐ adrenergic receptor-like GPCR polypeptide (or polynucleotide) or a test compound can be immobilized utilizing conjugation of biotin and streptavidin. Biotinylated human α₁ₐ adrenergic receptor-like GPCR polypeptides (or polynucleotides) or test compounds can be prepared from biotin-NHS(N-hydroxy-succinimide) using techniques well known in the art (*e*.*g*., biotinylation kit, Pierce Chemicals, Rockford, Ill.) and immobilized in the wells of streptavidin-coated 96 well plates (Pierce Chemical). Alternatively, antibodies which specifically bind to a human α₁ₐ adrenergic receptor-like GPCR polypeptide, polynucleotide, or a test compound, but which do not interfere with a desired binding site, such as the active site of the human α₁ₐ adrenergic receptor-like GPCR polypeptide, can be derivatized to the wells of the plate. Unbound target or protein can be trapped in the wells by antibody conjugation.

Methods for detecting such complexes, in addition to those described above for the GST-immobilized complexes, include immunodetection of complexes using antibodies which specifically bind to the human α₁ₐ adrenergic receptor-like GPCR polypeptide or test compound, enzyme-linked assays which rely on detecting an activity of the human α₁ₐ adrenergic receptor-like GPCR polypeptide, and SDS gel electrophoresis under non- reducing conditions.

Screening for test compounds which bind to a human α₁ₐ adrenergic receptor-like GPCR polypeptide or polynucleotide also can be carried out in an intact cell. Any cell which comprises a human α₁ₐ adrenergic receptor-like GPCR polypeptide or polynucleotide can be used in a cell-based assay system. A human α₁ₐ adrenergic receptor-like GPCR polynucleotide can be naturally occurring in the cell or can be introduced using techniques such as those described above. Binding of the test compound to a human α₁ₐ adrenergic receptor-like GPCR polypeptide or polynucleotide is determined as described above.

The specificity of binding of compounds showing affinity for the human α₁ₐ adrenergic receptor-like GPCR is shown by comparing affinity to membranes obtained from transfected cell lines that express the receptor and membranes from cell lines or tissues known to express other types of α *(e*.*g*., α _{1d}, α _{1b}) or beta adrenergic receptors. The specificity of binding of compounds showing affinity for the human α₁ₐ adrenergic receptor-like GPCR can be compared against the binding affinities to other types of alpha or beta adrenergic receptors. For example, the human alpha adrenergic receptor of the 1a subtype has been identified, cloned, and expressed (W094/08040 and WO 94/21660). Expression of cloned human α _{1d}, α _{1b} receptors and human α ₁ₐ adrenergic receptor-like GPCR and comparison of their binding properties with known selective antagonists provides a rational way for selection of compounds and discovery of new compounds with predictable pharmacological activities. Antagonism by these compounds of the human α₁ₐ adrenergic receptor-like GPCR subtype may be functionally demonstrated in anesthetized animals. These compounds may be used to increase urine flow without exhibiting orthostatic hypotensive effects.

Compounds identified using the screening methods described above may further be defined by counterscreening. This is accomplished according to methods known in the art using other receptors responsible for mediating diverse biological functions. See, *eg.*, W094/10989 and U.S. Patent 5,403,847. Compounds which are both selective amongst the various human α1 adrenergic receptor subtypes and which have low affinity for other receptors, such as the α2 adrenergic receptors, the β-adrenergic receptors, the muscarinic receptors, the serotonin receptors, and others are particularly preferred. The absence of these non-specific activities may be confirmed by using cloned and expressed receptors in an analogous fashion to the method disclosed herein for identifying compounds which have high affinity for the various human α1 adrenergic receptors. Furthermore, functional biological tests are used to confirm the effects of identified compounds as α1 a adrenergic receptor antagonists.

### Functional Assays

Test compounds can be tested for the ability to increase or decrease a biological effect of a GPCR polypeptide. Such biological effects can be determined using the functional assays described in the specific examples, below. Functional assays can be carried out after contacting either a purified GPCR polypeptide, a cell membrane preparation, or an intact cell with a test compound. A test compound which decreases a functional activity of a GPCR by at least about 10, preferably about 50, more preferably about 75, 90, or 100% is identified as a potential agent for decreasing GPCR activity. A test compound which increases GPCR activity by at least about 10, preferably about 50, more preferably about 75, 90, or 100% is identified as a potential agent for increasing GPCR activity.

One such screening procedure involves the use of melanophores which are transfected to express a GPCR polypeptide. Such a screening technique is described in WO 92/01810 published Feb. 6, 1992. Thus, for example, such an assay may be employed for screening for a compound which inhibits activation of the receptor polypeptide by contacting the melanophore cells which comprise the receptor with both the receptor ligand and a test compound to be screened. Inhibition of the signal generated by the ligand indicates that a test compound is a potential antagonist for the receptor, *i*.*e*., inhibits activation of the receptor. The screen may be employed for identifying a test compound which activates the receptor by contacting such cells with compounds to be screened and determining whether each test compound generates a signal, *i*.*e*., activates the receptor.

Other screening techniques include the use of cells which express a human GPCR polypeptide (for example, transfected CHO cells) in a system which measures extracellular pH changes caused by receptor activation (*see, e*.*g*., *Science 246*, 181-296, 1989). For example, test compounds may be contacted with a cell which expresses a human GPCR polypeptide and a second messenger response, *e*.*g*., signal transduction or pH changes, can be measured to determine whether the test compound activates or inhibits the receptor.

Another such screening technique involves introducing RNA encoding a human GPCR polypeptide into *Xenopus* oocytes to transiently express the receptor. The transfected oocytes can then be contacted with the receptor ligand and a test compound to be screened, followed by detection of inhibition or activation of a calcium signal in the case of screening for test compounds which are thought to inhibit activation of the receptor.

Another screening technique involves expressing a human GPCR polypeptide in cells in which the receptor is linked to a phospholipase C or D. Such cells include endothelial cells, smooth muscle cells, embryonic kidney cells, etc. The screening may be accomplished as described above by quantifying the degree of activation of the receptor from changes in the phospholipase activity.

Details of functional assays such as those described above are provided in the specific examples, below.

### GPCR Gene Expression

In another embodiment, test compounds which increase or decrease GPCR gene expression are identified. A GPCR polynucleotide is contacted with a test compound, and the expression of an RNA or polypeptide product of the GPCR polynucleotide is determined. The level of expression of appropriate mRNA or polypeptide in the presence of the test compound is compared to the level of expression of mRNA or polypeptide in the absence of the test compound. The test compound can then be identified as a modulator of expression based on this comparison. For example, when expression of mRNA or polypeptide is greater in the presence of the test compound than in its absence, the test compound is identified as a stimulator or enhancer of the mRNA or polypeptide expression. Alternatively, when expression of the mRNA or polypeptide is less in the presence of the test compound than in its absence, the test compound is identified as an inhibitor of the mRNA or polypeptide expression.

The level of GPCR mRNA or polypeptide expression in the cells can be determined by methods well known in the art for detecting mRNA or polypeptide. Either qualitative or quantitative methods can be used. The presence of polypeptide products of a GPCR polynucleotide can be determined, for example, using a variety of techniques known in the art, including immunochemical methods such as radioimmunoassay, Western blotting, and immunohistochemistry. Alternatively, polypeptide synthesis can be determined *in vivo*, in a cell culture, or in an *in vitro* translation system by detecting incorporation of labeled amino acids into a GPCR polypeptide.

Such screening can be carried out either in a cell-free assay system or in an intact cell. Any cell which expresses a GPCR polynucleotide can be used in a cell-based assay system. The GPCR polynucleotide can be naturally occurring in the cell or can be introduced using techniques such as those described above. Either a primary culture or an established cell line, such as CHO or human embryonic kidney 293 cells, can be used.

### Therapeutic Indications and Methods

GPCRs are ubiquitous in the mammalian host and are responsible for many biological functions, including many pathologies. Accordingly, it is desirable to find compounds and drugs which stimulate a GPCR on the one hand and which can inhibit the function of a GPCR on the other hand. For example, compounds which activate a GPCR may be employed for therapeutic purposes, such as the treatment of Parkinson's disease.

In general, compounds which inhibit activation of a GPCR can be used for a variety of therapeutic purposes, for example, for the treatment of psychotic and neurological disorders including schizophrenia, manic excitement, depression, delirium, dementia or severe mental retardation, dyskinesias, such as Huntington's disease or Tourett's syndrome, among others.

Obesity and overweight are defined as an excess of body fat relative to lean body mass. An increase in caloric intake or a decrease in energy expenditure or both can bring about this imbalance leading to surplus energy being stored as fat. Obesity is associated with important medical morbidities and an increase in mortality. The causes of obesity are poorly understood and may be due to genetic factors, environmental factors or a combination of the two to cause a positive energy balance. In contrast, anorexia and cachexia are characterized by an imbalance in energy intake versus energy expenditure leading to a negative energy balance and weight loss. Agents that either increase energy expenditure and/or decrease energy intake, absorption or storage would be useful for treating obesity, overweight, and associated comorbidities. Agents that either increase energy intake and/or decrease energy expenditure or increase the amount of lean tissue would be useful for treating cachexia, anorexia and wasting disorders.

This invention further pertains to the use of novel agents identified by the screening assays described above. Accordingly, it is within the scope of this invention to use a test compound identified as described herein in an appropriate animal model. For example, an agent identified as described herein (*e*.*g*., a modulating agent, an antisense nucleic acid molecule, a specific antibody, ribozyme, or a human α₁ₐ adrenergic receptor-like GPCR polypeptide binding molecule) can be used in an animal model to determine the efficacy, toxicity, or side effects of treatment with such an agent. Alternatively, an agent identified as described herein can be used in an animal model to determine the mechanism of action of such an agent Furthermore, this invention pertains to uses of novel agents identified by the above-described screening assays for treatments as described herein.

Effects of blocking human α₁ₐ adrenergic receptor-like GPCR include reduction of intra-ocular pressure, control of cardiac arrhythmias, and possibly a host of α_{1c} receptor mediated central nervous system events.

A reagent which affects human α₁ₐ adrenergic receptor-like GPCR activity can be administered to a human cell, either *in vitro* or *in vivo,* to reduce human α₁ₐ adrenergic receptor-like GPCR activity. The reagent preferably binds to an expression product of a human α₁ₐ adrenergic receptor-like GPCR gene. If the expression product is a protein, the reagent is preferably an antibody. For treatment of human cells *ex vivo*, an antibody can be added to a preparation of stem cells which have been removed from the body. The cells can then be replaced in the same or another human body, with or without clonal propagation, as is known in the art.

### EXAMPLE 1

### Detection of α₁ₐ adrenergic receptor-like GPCR activity

The polynucleotide of SEQ ID NO. 2 is inserted into the expression vector pCEV4 and the expression vector pCEV4-α₁ₐ adrenergic receptor-like GPCR polypeptide obtained is transfected into human embryonic kidney 293 cells. The cells are scraped from a culture flask into 5 ml of Tris HCl, 5 mM EDTA, pH 7.5, and lysed by sonication. Cell lysates are centrifuged at 1000 rpm for 5 minutes at 4°C. The supernatant is centrifuged at 30,000 x g for 20 minutes at 4°C. The pellet is suspended in binding buffer containing 50 mM Tris HCl, 5 mM MgSO₄, 1 mM EDTA, 100 mM NaCl, pH 7.5, supplemented with 0.1 % BSA, 2 µg/ml aprotinin, 0.5 mg/ml leupeptin, and 10 µg/ml phosphoramidon. Optimal membrane suspension dilutions, defined as the protein concentration required to bind less than 10 % of an added radioligand, i.e. ¹²⁵I-labeled norepinephrine, are added to 96-well polypropylene microtiter plates containing ligand, non-labeled peptides, and binding buffer to a final volume of 250 µl.

In equilibrium saturation binding assays, membrane preparations are incubated in the presence of increasing concentrations (0.1 nM to 4 nM) of ¹²⁵I ligand.

Binding reaction mixtures are incubated for one hour at 30°C. The reaction is stopped by filtration through GF/B filters treated with 0.5% polyethyleneimine, using a cell harvester. Radioactivity is measured by scintillation counting, and data are analyzed by a computerized non-linear regression program. Non-specific binding is defined as the amount of radioactivity remaining after incubation of membrane protein in the presence of 100 nM of unlabeled peptide. Protein concentration is measured by the Bradford method using Bio-Rad Reagent, with bovine serum albumin as a standard. The α₁ₐ adrenergic receptor-like GPCR activity of the polypeptide comprising the amino acid sequence of SEQ ID NO. 3 is demonstrated.

### EXAMPLE 2

### Radioligand binding assays

Human embryonic kidney 293 cells transfected with a polynucleotide which expresses human α₁ₐ adrenergic receptor-like GPCR are scraped from a culture flask into 5 ml of Tris HCl, 5 mM EDTA, pH 7.5, and lysed by sonication. Cell lysates are centrifuged at 1000 rpm for 5 minutes at 4°C. The supernatant is centrifuged at 30,000 x g for 20 minutes at 4°C. The pellet is suspended in binding buffer containing 50 mM Tris HCl, 5 mM MgSO₄, 1 mM EDTA, 100 mM NaCl, pH 7.5, supplemented with 0.1 % BSA, 2 µg/ml aprotinin, 0.5 mg/ml leupeptin, and 10 µg/ml phosphoramidon. Optimal membrane suspension dilutions, defined as the protein concentration required to bind less than 10 % of the added radioligand, i.e. norepinephrine, are added to 96-well polypropylene microtiter plates containing ¹²⁵I-labeled ligand or test compound, non-labeled peptides, and binding buffer to a final volume of 250 µl.

In equilibrium saturation binding assays, membrane preparations are incubated in the presence of increasing concentrations (0.1 nM to 4 nM) of ¹²⁵I-labeled ligand or test compound (specific activity 2200 Ci/mmol). The binding affinities of different test compounds are determined in equilibrium competition binding assays, using 0.1 nM ¹²⁵I- peptide in the presence of twelve different concentrations of each test compound.

Binding reaction mixtures are incubated for one hour at 30°C. The reaction is stopped by filtration through GF/B filters treated with 0.5% polyethyleneimine, using a cell harvester. Radioactivity is measured by scintillation counting, and data are analyzed by a computerized non-linear regression program.

Non-specific binding is defined as the amount of radioactivity remaining after incubation of membrane protein in the presence of 100 nM of unlabeled peptide.

Protein concentration is measured by the Bradford method using Bio-Rad Reagent, with bovine serum albumin as a standard. A test compound which increases the radioactivity of membrane protein by at least 15% relative to radioactivity of membrane protein which was not incubated with a test compound is identified as a compound which binds to a human α₁ₐ adrenergic receptor-like GPCR polypeptide.

### EXAMPLE 3

### Effect of a test compound on human α₁ₐ adrenergic receptor-like GPCR-mediated cyclic AMP formarion

Receptor-mediated inhibition of CANT formation can be assayed in host cells which express human α₁ₐ adrenergic receptor-like GPCR. Cells are plated in 96-well plates and incubated in Dulbecco's phosphate buffered saline (PBS) supplemented with 10 mM HEPES, 5 mM theophylline, 2 µg/ml aprotinin, 0.5 mg/ml leupeptin, and 10 µg/ml phosphoramidon for 20 minutes at 37°C in 5% CO2. A test compound is added and incubated for an additional 10 minutes at 37°C. The medium is aspirated, and the reaction is stopped by the addition of 100 mM HCl. The plates are stored at 4°C for 15 minutes. cAMP content in the stopping solution is measured by radioimmunoassay.

Radioactivity is quantified using a gamma counter equipped with data reduction software. A test compound which decreases radioactivity of the contents of a well relative to radioactivity of the contents of a well in the absence of the test compound is identified as a potential inhibitor of cAMP formation. A test compound which increases radioactivity of the contents of a well relative to radioactivity of the contents of a well in the absence of the test compound is identified as a potential enhancer of cAMP formation.

### EXAMPLE 4

### Effect of a test compound on the mobilization of intracellular calcium

Intracellular free calcium concentration can be measured by microspeetrofluorometry using the fluorescent indicator dye Fura-2/AM (Bush *et al*., *J. Neurochem. 57, 562*-74, 1991). Stably transfected cells are seeded onto a 35 mm culture dish containing a glass coverslip insert. Cells are washed with HBS , incubated with a test compound, and loaded with 100 µl of Fura-2/AM (10 µM) for 20-40 minutes. After washing with HBS to remove the Fura-2/AM solution, cells are equilibrated in HBS for 10-20 minutes. Cells are then visualized under the 40X objective of a Leitz Fluovert FS microscope.

Fluorescence emission is determined at 510 nM, with excitation wavelengths alternating between 340 nM and 380 nM. Raw fluorescence data are converted to calcium concentrations using standard calcium concentration curves and software analysis techniques. A test compound which increases the fluorescence by at least 15% relative to fluorescence in the absence of a test compound is identified as a compound which mobilizes intracellular calcium.

### EXAMPLE 5

### Effect of a test compound on phosphoinositide metabolism

Cells which stably express human α₁ₐ adrenergic receptor-like GPCR cDNA are plated in 96-well plates and grown to confluence. The- day before the assay, the growth medium is changed to 100 µl of medium containing 1% serum and 0.5 µCi ³H-myinositol. The plates are incubated overnight in a CO₂ incubator (5% CO₂ at 37°C). Immediately before the assay, the medium is removed and replaced by 200 µl of PBS containing 10 mM LiCl, and the cells are equilibrated with the new medium for 20 minutes. During this interval, cells also are equilibrated with antagonist, added as a 10 µl aliquot of a 20-fold concentrated solution in PBS.

The ³H-inositol phosphate accumulation from inositol phospholipid metabolism is started by adding 10 µl of a solution containing a test compound. To the first well 10 µl are added to measure basal accumulation. Eleven different concentrations of test compound are assayed in the following 11 wells of each plate row. All assays are performed in duplicate by repeating the same additions in two consecutive plate rows.

The plates are incubated in a CO₂ incubator for one hour. The reaction is terminated by adding 15 µl of 50% v/v trichloroacetic acid (TCA), followed by a 40 minute incubation at 4°C. After neutralizing TCA with 40 µl of 1 M Tris, the content of the wells is transferred to a Multiscreen HV filter plate (Millipore) containing Dowex AG1-X8 (200-400 mesh, formate form). The filter plates are prepared by adding 200 µl of Dowex AG1-X8 suspension (50% v/v, water:resin) to each well. The filter plates are placed on a vacuum manifold to wash or elute the resin bed. Each well is washed 2 times with 200 µl of water, followed by 2 x 200 µl of 5 mM sodium tetraborate/60 mM ammonium formate.

The ³H-IPs are eluted into empty 96-well plates with 200 µl of 1.2 M ammonium formate/0.1 formic acid. The content of the wells is added to 3 ml of scintillation cocktail, and radioactivity is determined by liquid scintillation counting.

### EXAMPLE 6

### Receptor Binding Methods

Standard Binding Assays. Binding assays are carried out in a binding buffer containing 50 mM HEPES, pH 7.4, 0.5% BSA, and 5 mM MgCl₂. The standard assay for radioligand binding to membrane fragments comprising human α₁ₐ adrenergic receptor-like GPCR polypeptides is carried out as follows in 96 well microtiter plates (*e*.*g*., Dynatech Immulon II Removawell plates). Radioligand is diluted in binding buffer+ PMSF/Baci to the desired cpm per 50 µl, then 50 µl aliquots are added to the wells. For non-specific binding samples, 5 µl of 40 µM cold ligand also is added per well. Binding is initiated by adding 150 µl per well of membrane diluted to the desired concentration (10-30 µg membrane protein/well) in binding buffer+ PMSF/Baci. Plates are then covered with Linbro mylar plate sealers (Flow Labs) and placed on a Dynatech Microshaker II. Binding is allowed to proceed at room temperature for 1-2 hours and is stopped by centrifuging the plate for 15 minutes at 2,000 x g. The supernatants are decanted, and the membrane pellets are washed once by addition of 200 µl of ice cold binding buffer, brief shaking, and recentrifugation. The individual wells are placed in 12 x 75 mm tubes and counted in an LKB Gammamaster counter (78% efficiency). Specific binding by this method is identical to that measured when free ligand is removed by rapid (3-5 seconds) filtration and washing on polyethyleneimine-coated glass fiber filters.

Three variations of the standard binding assay are also used.
1. Competitive radioligand binding assays with a concentration range of cold ligand vs. ¹²⁵ I-labeled ligand are carried out as described above with one modification. All dilutions of ligands being assayed are made in 40X PMSF/Baci to a concentration 40X the final concentration in the assay. Samples of peptide (5 µl each) are then added per microtiter well. Membranes and radioligand are diluted in binding buffer without protease inhibitors. Radioligand is added and mixed with cold ligand, and then binding is initiated by addition of membranes.
2. Chemical cross-linking of radioligand with receptor is done after a binding step identical to the standard assay. However, the wash step is done with binding buffer minus BSA to reduce the possibility of non-specific cross-linking of radioligand with BSA. The cross-linking step is carried out as described below.
3. Larger scale binding assays to obtain membrane pellets for studies on solubilization of receptor:ligand complex and for receptor purification are also carried out. These are identical to the standard assays except that (a) binding is carried out in polypropylene tubes in volumes from 1-250 ml, (b) concentration of membrane protein is always 0.5 mg/ml, and (c) for receptor purification, BSA concentration in the binding buffer is reduced to 0.25%, and the wash step is done with binding buffer without BSA, which reduces BSA contamination of the purified receptor.

### EXAMPLE 7

### Chemical Cross-Linking of Radioligand to Receptor

After a radioligand binding step as described above, membrane pellets are resuspended in 200 µl per microtiter plate well of ice-cold binding buffer without BSA. Then 5 µl per well of 4 mM N-5-azido-2-nitrobenzoyloxysuccinimide (ANB-NOS, Pierce) in DMSO is added and mixed. The samples are held on ice and UV-irradiated for 10 minutes with a Mineralight R-52G lamp (UVP Inc., San Gabriel, Calif.) at a distance of 5-10 cm. Then the samples are transferred to Eppendorf microfuge tubes, the membranes pelleted by centrifugation, supernatants removed, and membranes solubilized in Laemmli SDS sample buffer for polyacrylamide gel electrophoresis (PAGE). PAGE is carried out as described below. Radiolabeled proteins are visualized by autoradiography of the dried gels with Kodak XAR film and Dupont image intensifier screens.

### EXAMPLE 8

### Membrane Solubilization

Membrane solubilization is carried out in buffer containing 25 mM Tris , pH 8, 10% glycerol (w/v) and 0.2 mM CaCl₂ (solubilization buffer). The highly soluble detergents including Triton X-100, deoxycholate, deoxycholate:lysolecithin, CHAPS, and zwittergent are made up in solubilization buffer at 10% concentrations and stored as frozen aliquots. Lysolecithin is made up fresh because of insolubility upon freeze-thawing and digitonin is made fresh at lower concentrations due to its more limited solubility.

To solubilize membranes, washed pellets after the binding step are resuspended free of visible particles by pipetting and vortexing in solubilization buffer at 100,000 x g for 30 minutes. The supernatants are removed and held on ice and the pellets are discarded.

### EXAMPLE 9

### Assay of Solubilized Receptors

After binding of ¹²⁵I ligands and solubilization of the membranes with detergent, the intact R:L complex can be assayed by four different methods. All are carried out on ice or in a cold room at 4-10°C.).
1. Column chromatography (Knuhtsen *et al., Biochem. J. 254, 641-647,* 1988). Sephadex G-50 columns (8 x 250 mm) are equilibrated with solubilization buffer containing detergent at the concentration used to solubilize membranes and 1 mg/ml bovine serum albumin. Samples of solubilized membranes (0.2-0.5 ml) are applied to the columns and eluted at a flow rate of about 0.7 ml/minute. Samples (0.18 ml) are collected. Radioactivity is determined in a gamma counter. Void volumes of the columns are determined by the elution volume of blue dextran. Radioactivity eluting in the void volume is considered bound to protein. Radioactivity eluting later, at the same volume as free ¹²⁵I ligands, is considered non-bound.
2. Polyethyleneglycol precipitation (Cuatrecasas, *Proc. Natl. Acad. Sci. USA 69,* 318-322, 1972). For a 100 µl sample of solubilized membranes in a 12 x 75 mm polypropylene tube, 0.5 ml of 1% (w/v) bovine gamma globulin (Sigma) in 0.1 M sodium phosphate buffer is added, followed by 0.5 ml of 25% (w/v) polyethyleneglycol (Sigma) and mixing. The mixture is held on ice for 15 minutes. Then 3 ml of 0.1 M sodium phosphate, pH 7.4, is added per sample. The samples are rapidly (1-3 seconds) filtered over Whatman GF/B glass fiber filters and washed with 4 ml of the phosphate buffer. PEG-precipitated receptor : ¹²⁵ I-ligand complex is determined by gamma counting of the filters.
3. GFB/PEI filter binding (Bruns *et al., Analytical Biochem*. *132,* 74-81, 1983). Whatman GF/B glass fiber filters are soaked in 0.3% polyethyleneimine (PEI, Sigma) for 3 hours. Samples of solubilized membranes (25-100 µl) are replaced in 12 x 75 mm polypropylene tubes. Then 4 ml of solubilization buffer without detergent is added per sample and the samples are immediately filtered through the GFB/PEI filters (1-3 seconds) and washed with 4 ml of solubilization buffer. CPM of receptor : ¹²⁵ I-ligand complex adsorbed to filters are determined by gamma counting.
4. Charcoal/Dextran (Paul and Said, *Peptides 7[Suppl. 1]*,147-149, 1996). Dextran T70 (0.5 g, Pharmacia) is dissolved in 1 liter of water, then 5 g of activated charcoal (Norit A, alkaline; Fisher Scientific) is added. The suspension is stirred for 10 minutes at room temperature and then stored at 4°C. until use. To measure R:L complex, 4 parts by volume of charcoal/dextran suspension are added to 1 part by volume of solubilized membrane. The samples are mixed and held on ice for 2 minutes and then centrifuged for 2 -minutes at 11,000 x g in a Beckman microfuge. Free radioligand is adsorbed charcoal/dextran and is discarded with the pellet. Receptor : ¹²⁵ I-ligand complexes remain in the supernatant and are determined by gamma counting.

### EXAMPLE 10

### Receptor Purification

Binding of biotinyl-receptor to GH₄ Cl membranes is carried out as described above. Incubations are for 1 hour at room temperature. In the standard purification protocol, the binding incubations contain 10 nM Bio-S29. ¹²⁵I ligand is added as a tracer at levels of 5,000-100,000 cpm per mg of membrane protein. Control incubations contain 10 µM cold ligand to saturate the receptor with non-biotinylated ligand.

Solubilization of receptor.ligand complex also is carried out as described above, with 0.15% deoxycholaxe:lysolecithin in solubilization buffer containing 0.2 mM MgCl₂, to obtain 100,000 x g supernatants containing solubilized R:L complex.

Immobilized streptavidin (streptavidin cross-linked to 6% beaded agarose, Pierce Chemical Co.; "SA-agarose") is washed in solubilization buffer and added to the solubilized membranes as 1/30 of the final volume. This mixture is incubated with constant stirring by end-over-end rotation for 4-5 hours at 4-10 °C. Then the mixture is applied to a column and the non-bound material is washed through. Binding of radioligand to SA-agarose is determined by comparing. cpm in the 100,000 x g supernatant with that in the column effluent after adsorption to SA-agarose. Finally, the column is washed with 12-15 column volumes of solubilization buffer+0.15% deoxycholate:lysolecithin +1/500 (vol/vol) 100 x 4pase.

The streptavidin column is eluted with solubilization buffer+0.1 mM EDTA+0.1 mM EGTA+0.1 mM GTP-gamma-S (Sigma)+0.15% (wt/vol) deoxycholate:lysolecithin +1/1000 (vol/vol) 100.times.4pase. First, one column volume of elution buffer is passed through the column and flow is stopped for 20-30 minutes. Then 3-4 more column volumes of elution buffer are passed through. All the eluates are pooled.

Eluates from the streptavidin column are incubated overnight (12-15 hours) with immobilized wheat germ agglutinin (WGA agarose, Vector Labs) to adsorb the receptor via interaction of covalently bound carbohydrate with the WGA lectin. The ratio (vol/vol) of WGA-agarose to streptavidin column eluate is generally 1:400. A range from 1:1000 to 1:200 also can be used. After the binding step, the resin is pelleted by centrifugation, the supernatant is removed and saved, and the resin is washed 3 times (about 2 minutes each) in buffer containing 50 mM HEPES, pH 8, 5 mM MgCl₂, and 0.15% deoxycholate:lysolecithin. To elute the WGA-bound receptor, the resin is extracted three times by repeated mixing (vortex mixer on low speed) over a 15-30 minute period on ice, with 3 resin columns each time, of 10 mM N-N'-N"-triacetylchitotriose in the same HEPES buffer used to wash the resin. After each elution step, the resin is centrifuged down and the supernatant is carefully removed, free of WGA-agarose pellets. The three, pooled eluates contain the final, purified receptor. The material non-bound to WGA contain G protein subunits specifically eluted from the streptavidin column, as well as non-specific contaminants. All these fractions are stored frozen at -90°C.

### EXAMPLE 11

### Identification of test compounds that bind to human α₁ₐ adrenergic receptor-like GPCR polypeptides

Purified human α₁ₐ adrenergic receptor-like GPCR polypeptides comprising a glutathione-S-transferase protein and absorbed onto glutathione-derivatized wells of 96-well microtiter plates are contacted with test compounds from a small molecule library at pH 7.0 in a physiological buffer solution. Human α₁ₐ adrenergic receptor-like GPCR polypeptides comprise an amino acid sequence shown in SEQ ID NO. 3. The test compounds comprise a fluorescent tag. The samples are incubated for 5 minutes to one hour. Control samples are incubated in the absence of a test compound.

The buffer solution containing the test compounds is washed from the wells. Binding of a test compound to a human α₁ₐ adrenergic receptor-like GPCR polypeptide is detected by fluorescence measurements of the contents of the wells. A test compound which increases the fluorescence in a well by at least 15% relative to fluorescence of a well in which a test compound was not incubated is identified as a compound which binds to a human α₁ₐ adrenergic receptor-like GPCR polypeptide.

### EXAMPLE 12

### Selective binding assays

Membranes prepared from stably transfected human α_{1d} and α_{1b} cell lines (ATCC CRL 11138 and CRL 11139, respectively) are used to identify compounds that selectively bind to the human α ₁ₐ adrenergic receptor-like GPCR. These competition binding reactions (total volume=200 µl) contain 50 mM Tris-HCl pH. 7.4, 5 mM EDTA, 150 mM NaCl, 100 pM ¹²⁵ I-HEAT, membranes prepared from cell lines transfected with the respective α1 subtype expression plasmid, and increasing amounts of unlabeled ligand. Reactions are incubated at room temperature for one hour with shaking. Reactions are filtered onto Whatman GF/C glass fiber filters with an Inotec 96 well cell harvester. Filters are washed three times with ice cold buffer and bound radioactivity is determined (Ki).

### EXAMPLE 13

### Exemplary functional assays

In order to confirm the specificity of compounds for the human α ₁ₐ adrenergic receptor-like GPCR and to define the biological activity of the compounds, the following functional tests may be performed:
1. *In vitro* Rat, Dog and Human Prostate and Dog Urethra
   Taconic Farms Sprague-Dawley male rats, weighing 250-400 grams are sacrificed by cervical dislocation under anesthesia (methohexital; 50 mg/kg, i.p.). An incision is made into the lower abdomen to remove the ventral lobes of the prostate. Each prostate removed from a mongrel dog is cut into 6-8 pieces longitudinally along the urethra opening and stored in ice-cold oxygenated Krebs solution overnight before use if necessary. Dog urethra proximal to prostate is cut into approximately 5 mm rings, the rings are then cut open for contractile measurement of circular muscles. Human prostate chips from transurethral surgery of benign prostate hyperplasia are also stored overnight in ice-cold Krebs solution if needed.
   The tissue is placed in a Petri dish containing oxygenated Krebs solution (NaCl, 118 mM; KCI, 4.7 mM; CaCl₂, 2.5 mM; KH₂PO₄, 1.2 mM; MgSO₄, 1.2.mM; NaHCO₃, 2.0 mM; dextrose, 11 mM) warmed to 37°C. Excess lipid material and connective tissue are carefully removed. Tissue segments are attached to glass tissue holders with 4-0 surgical.silk and placed in a 5 ml jacketed tissue bath containing Krebs buffer at 37 °C., bubbled with 5% CO₂/95% O₂. The tissues are connected to a Statham-Gould force transducer; 1 gram (rat, human) or 1.5 gram (dog) of tension is applied and the tissues are allowed to equilibrate for one hour. Contractions are recorded on a Hewlett-Packard 7700 series strip chart recorder.
   After a single priming dose of 3 µM (for rat), 10 µM (for dog) and 20 µM (for human) of phenylephrine, a cumulative concentration response curve to an agonist is generated; the tissues are washed every 10 minutes for one hour. Vehicle or antagonist is added to the bath and allowed to incubate for one hour, then another cumulative concentration response curve to the agonist is generated.
   EC₅₀ values are calculated for each group using GraphPad Inplot software. pA2 (approximately log Kb) values are obtained from Schild plot when three or more concentrations are tested.
2. Measurement of Intra-Urethral Pressure in Anesthetized Dogs
   The following model is used to measure adrenergically mediated changes in intra-urethral pressure and arterial pressure in anesthetized dogs in order to evaluate the efficacy and potency of selective human α₁ₐ adrenergic receptor-like GPCR antagonists. Male mongrel dogs (7-12 kg) are used in this study. The dogs are anesthetized with pentobarbital sodium (35 mg/kg, i.v. plus 4 mg/kg/hr iv infusion). An endotracheal tube is inserted and the animal ventilated with room air using a Harvard instruments positive displacement large animal ventilator. Catheters (PE 240 or 260) are placed in the aorta via the femoral artery and vena cava via the femoral veins (2 catheters, one in each vein) for the measurement of arterial pressure and the administration of drugs, respectively. A supra-pubic incision about 1/2 inch lateral to the penis is made to expose the ureters, bladder and urethra. The urethers are ligated and cannulated so that urine flows freely into beakers. The dome of the bladder is retracted to facilitate dissection of the proximal and distal urethra. Umbilical tape is passed beneath the urethra at the bladder neck and another piece of umbilical tape is placed under the distal urethra approximately 1-2 cm distal to the prostate. The bladder is incised and a Millar micro-tip pressure transducer is advanced into the urethra. The bladder incision is sutured with 2-0 or 3-0 silk (purse-string suture) to hold the transducer. The tip of the transducer is placed in the prostatic urethra and the position of the Millar catheter is verified by gently squeezing the prostate and noting the large change in urethral pressure.
   Phenylephrine, an α1 adrenergic agonist, is administered (0.1-100 µg/kg, iv; 0.05 ml/kg volume) in order to construct dose response curves for changes in intra-urethral and arterial pressure. Following administration of increasing doses of an alpha adrenergic antagonist (or vehicle), the effects of phenylephrine on arterial pressure and intra-urethral pressure are re-evaluated. Four or five phenylephrine dose-response curves are generated in each animal (one control, three or four doses of antagonist or vehicle). The relative antagonist potency on phenylephrine induced changes in arterial and intra-urethral pressure are determined by Schild analysis. The family of averaged curves are fit simultaneously (using ALLFIT software package) with a four parameter logistic equation constraining the slope, minimum response, and maximum response to be constant among curves. The dose ratios for the antagonist doses (rightward shift in the dose-response curves from control) are calculated as the ratio of the ED₅₀ s for the respective curves. These dose-ratios are then used to construct a Schild plot and the Kb (expressed as µg/kg, iv) determined. The Kb (dose of antagonist causing a 2-fold rightward shift of the phenylephrine dose-response curve) is used to compare the relative potency of the antagonists on inhibiting phenylephrine responses for intra-urethral and arterial pressure. The relative selectivity is calculated as the ratio of arterial pressure and intra-urethral pressure, Kbs. Effects of the α1 antagonists on baseline arterial pressure are also monitored. Comparison of the relative antagonist potency on changes in arterial pressure and intra-urethral pressure provide insight as to whether the alpha receptor subtype responsible for increasing intra-urethral pressure is also present in the systemic vasculature. According to this method, one is able to confirm the selectivity of α₁ₐ adrenergic receptor antagonists that prevent the increase in intra-urethral pressure to phenylephrine without any activity at the vasculature.

### EXAMPLE 14

### Identification of a test compound which decreases human α₁ₐ adrenergic receptor-like GPCR gene expression

A test compound is administered to a culture of human gastric cells and incubated at 37°C for 10 to 45 minutes. A culture of the same type of cells incubated for the same time without the test compound provides a negative control.

RNA is isolated from the two cultures as described in Chirgwin *et al., Biochem. 18,* 5294-99, 1979). Northern blots are prepared using 20 to 30 µg total RNA and hybridized with a ³²P-labeled human α₁ₐ adrenergic receptor-like GPCR-specific probe at 65°C in Express-hyb (CLONTECH). The probe comprises at least 11 contiguous nucleotides selected from the complement of SEQ ID NO.1. A test compound which decreases the human α₁ₐ adrenergic receptor-like GPCR-specific signal relative to the signal obtained in the absence of the test compound is identified as an inhibitor of human α₁ₐ adrenergic receptor-like GPCR gene expression.

### EXAMPLE 15

### Treatment of obesity with a reagent which specifically binds to a GPCR gene product

Synthesis of antisense human α₁ₐ adrenergic receptor-like GPCR oligonucleotides comprising at least 11 contiguous nucleotides selected from the complement of SEQ ID NO. 2 is performed on a Pharmacia Gene Assembler series synthesizer using the phosphoramidite procedure (Uhlmann *et al., Chem. Rev. 90*, 534-83, 1990).

Following assembly and deprotection, oligonucleotides are ethanol-precipitated twice, dried, and suspended in phosphate-buffered saline (PBS) at the desired concentration. Purity of these oligonucleotides is tested by capillary gel electrophoreses and ion exchange HPLC. Endotoxin levels in the oligonucleotide preparation are determined using the *Limulus* Amebocyte Assay (Bang, *Biol. Bull. (Woods Hole, Mass.) 105,* 361-362, 1953).

The antisense oligonucleotides are administered to a patient with obesity. The severity of the patient's obesity decreases.

### EXAMPLE 16

### Tissue specific expression

### PCR analysis using cDNA phage libraries from human tissues

Human cDNA phage libraries (Stratagene) were purchased in a "human tissue panel IA" as described in Table1. One-half µl of each library-purchased sample were used as template in PCR analysis regardless the title (phage/ml) for non-quantitative expression analysis. In addition, a positive control PCR reaction was performed with about 20 ng of human genomic DNA as template, and a negative control was performed with no template.

The standard PCR procedures were as indicated by Perkin Elmer. The PCR protocol was as follows:
Primers:
PCR reaction mix:

| | |
|---|---|
| 0.5µl | template |
| 1 x | Gold PCR Buffer (Perkin Elmer) |
| 0.2 mM | dNTPs (Pharmacia) |
| 1.5mM | MgCl₂ (Perkin Elmer) |
| 0.5 µM | primer A |
| 0.5 µM | primer B |
| 2.5 U | AmpliTaq Gold DNA Polymerase (Pezkin Elmer) |
| to 25 µl final reaction volume with sterile H₂O. | |

The amplification protocol was performed in Perkin Elmer 9700 thermocycler:
1 X the following step:
   - pre PCR: 9' at 94°C
40 times the following steps:
   - denaturation: 30" at 94°C
   - annealing: 1' at 56°C
   - elongation: 30" at 72°C

Expected leagth of specific PCR product: 560bp.

Amplification products were analyzed by electrophoresis on a 2% agarose (SeaKem LE agarose, FMC bioproducts) gel in 1XTAE running buffer following standard procedure, as described by Maniatis *et al*. PCR amplification products of the expected size were detectable in the positive control (human genomic DNA) and in lanes corresponding to the Brain (Corpus Striatum) cDNA phage library.

To check PCR product identity, a mixture of the amplification products obtained was used for restriction analysis with the enzyme Apal (BioLabs) according to the manufacturer's instructions. Restriction fragments were analysed by electrophoresis on 2% agarose (SeaKem LE agarose, FMC bioproducts) gel in 1 X TAE running buffer following standard procedure, as described by Maniatis *et al*. Restriction by ApaI produced two fragments of the expected size (about 240bp and 330bp).

### PCR analysis using cDNAs from human tissues

Human QUICK-clone cDNAs (Clontech) were ordered in a "human tissue panel IB" (unless differently specified) as described in Table 2. One-half µl of each cDNA purchased sample were used as template in PCR for non quantitative expression analysis. In addition, a positive control PCR reaction was performed with about 20 ng of human genomic DNA as template, and a negative control was performed with no template.

The experimental protocol was as described above.

PCR amplification products of the expected size as shown in Fig. 9 were detectable in the positive control (human genomic DNA) and in lanes corresponding to the following cDNAs:
- Whole Brain
- Brain (hippocampus)
- Brain (cerebellum)
- Spinal cord -very faint amplification product
- Bladder
- Prostate
- Adrenal gland - very faint amplification product

**Table 1**

| Library | Description | Catalog |
|---|---|---|
| Brain(corpus striatum) | Caudate and putamen, males, 57 & 63 years old | 936213 |
| Brain (fetal) | Male and female, Caucasian | 937227 |
| Brain (frontal cortex) | Female, 85 years old | 936212 |
| Brain (substantia nigra) | Male and female, 60 years old | 936210 |
| Brain (occipital cortex) | Female, 85 years old | 936211 |
| Brain stem | Female,2 years old | 935206 |
| Bronchial muscle | Human bronchial/tracheal smooth muscle primary cells | 780032 |
| Coronary | Coronary artery endothelial primary cells | 780025 |
| Coronary | Coronary artery smooth muscle primary cells | 780029 |
| Endothelial | Microvascular endothelial primary cells | 780028 |
| Heart | 12 pooled, 19-50 years old, male/female Caucasian | 937257 |
| Kidney | 8 pooled, whole kidney from 24-55 years old, male/female, Caucasian | 937250 |
| Liver | Normal,38 years old, Caucasian | 937241 |
| Lung | Male, 72 years old, normal | 937210 |
| Muscle (skeletal) | Female, 19 years old | 936215 |
| Ovary | Normal, 49 years old, Caucasian | 937217 |
| Pulmonary artery endothelial | Pulmonary artery endothelial primary cells | 780027 |
| Umbilical artery endothelial cells | Umbilical artery endothelial cells | 780023 |
| Whole Brain | Whole brain 60 year | HL5018t |
| Spinal chord | Whole, pooled from 26 male/female, 16-75 years | HL5001b |

### EXAMPLE 17

### Cloning of full length cDNA

Full length cDNA was obtained by PCR using human genomic DNA as template. Standard PCR procedure was as indicated by Perkin Elmer.
PCR protocol was as follows:
Primers:
PCR reaction mix:

| | |
|---|---|
| 0.5 µl | genomic DNA 50ng |
| 5 µl | 10x Stratagene Cloned Pfu Buffer, |
| 0.2 mM | dNTPs (Pharmacia) |
| 0.5 µM | primer C |
| 0.5 µM | primer D |
| 2.5 U | Pfu DNA Polymerase ( Stratagene, Catalogue 600154) |
| to 50 µl final reaction volume with sterile H₂O. | |

The amplification protocol was performed in Perkin Elmer 9700 thermocycler:
1 time the following step:
   - pre PCR: 4' at 94°C
30 times the following steps:
   - denaturation: 1' at 94°C
   - annealing: 1' at 56°C
   - elongation: 1'30"at 72°C
   - 1 time the following step:: 7' at 72°C

The expected length of specific PCR product was 1694 bp.

PCR product length was controlled by electrophoresis on 1% agarose (SeaKem LE agarose, FMC bioproducts) gel in 1XTAE running buffer following standard procedure, as described by Maniatis *et al*. The electrophoretic band of the expected size was excised from the gel and purified by quiaquik gel extraction kit (Quiagen) following instructions.

A nested PCR reaction was done using the DNA obtained from the first PCR reaction as template.

Standard PCR procedure was as indicated by Perkin Elmer.

PCR protocol was as follows:
Primers:
PCR reaction mix:

| | |
|---|---|
| 0.5 µl | template |
| 5 µl | 10x Stratagene Cloned Pfu Buffer, |
| 0.2 mM | dNTPs (Pharmacia) |
| 0.5 µM | primer E |
| 0.5 µM | primer F |
| 2.5 U | Pfu DNA Polymerase (Stratagene, Catalogue 600154) |
| to 50 µl final reaction volume with sterile H₂O. | |

The amplification protocol was performed in Perkin Elmer 9700 thermocycler:
1 time the following step:
   - pre PCR: 4' at 94°C
30 times the following steps:
   - denaturation: 1' at 94°C
   - annealing: 1' at 56°C
   - elongation: 1'30" at 72°C
   - 1 time the following step:: 10' at 72°C

After the PCR reaction, the PCR product was purified using a purification column (Quiaquick PCR Purification kit, catalogue 28104 Quiagen). After this step an "A-Tailing" reaction was performed by adding 0.2mM dATP (Pharmacia) and 2.5U AmpiTaq Gold DNA polymerase (Perkin Elmer) to the PCR product and incubating in a Perkin Elmer 9700 thermocycler:
10' at 72°C

The purified PCR product was cloned in pCR II TOPO™ vector (catalogue 45-0640, Invitrogen) with Rapid DNA Ligation Kit, (catalogue 1635-379, Roche Diagnostics) following standard procedure and manufacturer's instructions.

A recombinant clone, named #96pCRIITOPO, was selected and sequenced. Sequencing revealed that the recombinant plasmid contains the full length ORF as deposited in databank, with no mutations. See SEQ ID NOS. 7 and 8.

### EXAMPLE 18

### Subcloning of full length cDNA in a mammalian expression vector

The pIRES2-EGFP (Catalogue 6029-1, CLONTECH Laboratories, Inc.) vector was selected for expression in mammalian cells. This vector contains the internal ribosome entry site of the encephalomyocarditis virus (ECMV) between the Multiple Cloning Sites and the enhanced green fluorescent protein (EGFP) coding region. This allows the gene of interest (cloned into the MCS) and the EGFP gene to be transcribed in a single bicistronic mRNA and therefore to use it for a highly efficient selection (by flow cytometry) of transfected cells.

The EcoRI-BamHI restriction fragment of human α_{1A} adrenergic receptor-like GPCR-pGRIITOPO was inserted in oRI-BamHI digested pIRES2-EGFP following standard procedure. Plasmid DNA was isolated from transformants and examined by restriction analysis for the presence of the correct fragment and correct 5'-3' orientation.

### EXAMPLE 19

### Quantitative analysis of relative expression of α₁ₐ adrenergic receptor-like GPCR in human tissues

Quantitative expression profiling was performed by the form of quantitative PCR analysis called "kinetic analysis" firstly described in Higuchi *et al*., 1992 and Higuchi et aL, 1993. The principle is that at any given cycle within the exponential phase of PCR, the amount of product is proportional to the initial number of template copies.

If the amplification is performed in the presence of an internally quenched fluorescent oligonucleotide (TaqMan probe) complementary to the target sequence, the probe is cleaved by the 5'-3' endonuclease activity of.Taq DNA polymerase and a fluorescent dye released in the medium (Holland et al.). Since the fluorescence emission will increase in direct proportion to the amount of the specific amplified product, the exponential growth phase of PCR product can be detected and used to determine the initial template concentration (Heid et aL, 1996, and Gibson et al., 1996).

The amplification of an endogenous control can be performed to standardize the amount of sample RNA added to a reaction. In this kind of experiments the control of choice is the 18S ribosomal RNA. Since reporter dyes with differing emission spectra are available, the target and the endogenous control can be independently quantified in the same tube if probes labeled with different dyes are used.

All "real time PCR" measurements of fluorescence were made in the ABI Prism 7700 Sequence detector System (PE Applied Biosystems, Foster City, CA).

### References

- Higuchi, R, Dollinger, G., Walsh, P.S. and Griffith, R 1992. Simultaneous amplification and detection of specific DNA sequences. *BioTechnology* 10:413-417.
- Higuchi, R., Fockler, C., Dollinger, G. and Watson, R. 1993. Kinetic PCR analysis: real-time monitoring of DNA amplification reactions.. *BioTechnology* 11:1026-1030.
- Holland, P.M., Abramson, R.D., Watson, R. and Gelfand, D.H. 1991. Detection of specific polymerase chain reaction product by utilizing the 5'-3' exonuclease activity of *Thermus aquaticus* DNA polymerase. *Proc. Natl. Acad Sci.* 88:7276-7280.
- Held, C., Stevens, J., Livak, K. And Williams, P.M. 1996. Real time quantitative PCR. *Genome Res.* 6:986-994.
- Gibson, U.E., Heid, C.A. and Williams, P.M. 1996. A novel method for real time quantitative RT-PCR. *Genome Res.* 6: 995-1001.

### cDNA preparation

The total RNAs used for expression quantification are listed in Table 3 along with their sources. Fifty µgs of each RNA were treated with DNase I for 1 hour at 37°C in the following reaction mix:

| | |
|---|---|
| DNase I, RNase-free (Roche Diagnostics, Germany) | 0.2 U/µL |
| RNase inhibitor (PE Applied Biosystems, CA) | 0.4 U/µL |
| Tris-HCl pH 7.9 | 10mM |
| MgCl₂ | 10mM |
| NaCl | 50mM |
| DTT | 1mM |

After incubation, RNA was extracted once with 1 volume of phenol:chloroform:isoamyl alcohol (24:24:1) and once with chloroform, and precipitated with 1/10 volume of NaAcetate 3M pH5.2 and 2 volume ethanol. After spectrophotometric quantification, each sample has been reverse transcribed with the TaqMan Reverse Transcription Reagents (PE Applied Biosystems, CA) accordingly to purchaser protocol. RNA final concentration in the reaction mix was 200ng/µL. Reverse transcription was made with 2.5µM of random hexamers.

### TaqMan quantitative analysis

Specific primers and probe were designed accordingly to PE Applied Biosystems recommendations and are listed below: where FAM = 6-carboxy-fluorescein
and TAMRA = 6-carboxy-tetramethyl-rhodamine.
The expected length of the PCR product was 63bp.

Quantification experiments were performed on 50 ng of reverse transcribed RNA from each sample. Each determination was done in triplicate.

Total cDNA content was normalized with the simultaneous quantification (multiplex PCR) of the 18S ribosomal RNA by use of the Pre-Developed TaqMan Assay Reagents (PDAR) Control Kit (PE Applied Biosystems, CA).
Assay reaction mix was as follows:

| | final |
|---|---|
| TaqMan Universal PCR Master Mix (2x) (PE Applied Biosystems, CA) | 1x |
| PDAR control - 18S RNA (20x | 1x |
| Forward primer | 900nM |
| Reverse primer | 300nM |
| Probe | 200nM |
| cDNA | 10ng |
| Water | to 25µL |

PCR conditions were:
1 time the allowing steps:
   - pre PCR: 2' at 50°C
   10' at 95°C
40 times the following steps:
   - denaturation: 15" at 95°C
   - annealing/extension: 1' at 60°C

The experiment was performed on an ABI Prism 7700 Sequence Detector (PE Applied Biosystems, CA). At the end of the run, fluorescence data acquired during PCR were processed as described in the ABI Prism 7700 user's manual in order to achieve better background subtraction as well as signal linearity with the starting target quantity.
Results are shown in Fig. 10, 11 and 12.

**Table 1**

| (see Fig. 10) | |
|---|---|
| **RNA** | **Purch.&#catalog** |
| h. Fetal Brain | Clontech (CA) 640191 |
| h. Brain | OriGene (MD) HT1001 |
| h. Muscle | OriGene (MD) HT1008 |
| h. Heart | OriGene (MD) HT1002 |
| h. Lung | OriGene (MD) HT1009 |
| h. Kidney | OriGene (MD) HT1003 |
| h. Liver | OriGene (MD) HT1005 |
| h. Thymus | Clontech (CA) 640281 |
| h. Testis | OriGene (MD) HT1011 |
| h. Colon | OriGene (MD) HT1015 |
| h. Placenta | OriGene (MD) HT1013 |

**Table 2**

| (see Fig. 11) | |
|---|---|
| **RNA** | **Purch.&#catalog** |
| h. Fetal Liver | Clontech (CA) 640181 |
| h. Bladder | Invitrogen (CA) D602001 |
| h. Prostate | Clontech (CA) 640381 |
| h. Adrenal Gland | Clontech (CA) 640161 |
| h. Spleen | OriGene (MD) HT1004 |
| h. Hypertrophic Prostate | from autopsy |
| h. Prostate | from autopsy |

**Table 3**

| **cDNA** | **Description** | **Catalog** |
|---|---|---|
| H. Brain whole | from 2 female, years 16 and 36 | 7187-1 |
| H. Brain, cerebral cortex | from 1 male, 66 years. | 7110-1 |
| H. Brain, cerebellum | from 11 male/female, 16-70 years. | 7120-1 |
| H. Brain, hippocampus | From 25 male/female, 16-70 years. | 7169-1 |
| H. Spinal Cord | from 69 male/female, years. 22-70 | 7163-1 |
| H. Heart | from 7 male/female, years. 20-78 | 7121-1 |
| H. Kidney | from 8 male/female, years. 24-55 | 7112-1 |
| H. Liver | from 1 male/1 female, years. 44 and 45 | 7113-1 |
| Fetal Liver | | 640181 |
| H.Bladder | | D602001 Invitrogen |
| H. Prostate | | 640381 |
| H. Adrenal gland | | 640161 |
| H. Spleen | | HT1004 Origene |

## Claims

1. Use of a host cell comprising a vector, said vector comprising a polynucleotide, said polynucloetide encoding
(i) an amino acid sequence comprising SEQ ID NO:3, or
(ii) an amino acid sequence which is at least 90% identical to SEQ ID NO:3,
for the screening for substances, useful in the treatment of a Central Nervous System disorder.

2. Use of a polypeptide comprising
(i) an amino acid sequence of SEQ ID NO:3, or
(ii) an amino acid sequence which is at least 90% identical to SEQ ID NO:3,
for the screening for substances, useful in the treatment of a Central Nervous System disorder.

3. Use of claim 1 or 2, wherein the Central Nervous System disorder is pain.

## Patentansprüche

1. Verwendung einer einen Vektor umfassenden Wirtszelle, wobei der Vektor ein Polynucleotid umfasst, wobei das Polynucleotid
(i) für eine Seq.-ID Nr. 3 umfassende Aminosäuresequenz oder
(ii) für eine Aminosäuresequenz, die zu zumindest 90 % mit Seq.-ID Nr. 3 identisch ist, kodiert,
zum Screenen auf Substanzen, die bei der Behandlung einer Störung des Zentralnervensystems von Nutzen sind.

2. Verwendung eines Polypeptids, das
(a) eine Aminosäuresequenz der Seq.-ID Nr. 3 oder
(b) eine Aminosäuresequenz, die zu zumindest 90 % mit Seq.-ID Nr. 3 identisch ist, umfasst,
zum Screenen auf Substanzen, die bei der Behandlung einer Störung des Zentralnervensystems von Nutzen sind.

3. Verwendung nach Anspruch 1 oder 2, worin die Störung des Zentralnervensystems Schmerz ist.

## Revendications

1. Utilisation d'une cellule hôte comprenant un vecteur, ledit vecteur comprenant un polynucléotide, ledit polynucléotide codant pour :
(i) une séquence d'aminoacides comprenant la SEQ ID N°3, ou
(ii) une séquence d'aminoacides qui est identique à au moins 90 % à la SEQ ID N°3,
pour la sélection de substances utiles dans le traitement d'un trouble du système nerveux central.

2. Utilisation d'un polypeptide comprenant :
(i) une séquence d'aminoacides de la SEQ ID N°3, ou
(ii) une séquence d'aminoacides qui est identique à au moins 90 % à la SEQ ID N°3,
pour la sélection de substances utiles dans le traitement d'un trouble d'un système nerveux central.

3. Utilisation suivant la revendication 1 ou 2, dans laquelle le trouble du système nerveux central est la douleur.
